(19) Europäisches Patentamt
European Patent Office
Office européen des brevets

(11) Veröffentlichungsnummer : **0 011 591**
**B1**

(12) # EUROPÄISCHE PATENTSCHRIFT

(45) Veröffentlichungstag der Patentschrift : **22.09.82**

(51) Int. Cl.³ : **C 07 C177/00**, C 07 C 69/732, C 07 C 59/46, A 61 K 31/557

(21) Anmeldenummer : **79710099.7**

(22) Anmeldetag : **18.10.79**

(54) **Neue Prostanderivate, ihre Herstellung und sie enthaltende Arzneimittel.**

(30) Priorität : **19.10.78 DE 2845770**

(43) Veröffentlichungstag der Anmeldung : **28.05.80 (Patentblatt 80/11)**

(45) Bekanntmachung des Hinweises auf die Patenterteilung : **22.09.82 Patentblatt 82/38**

(84) Benannte Vertragsstaaten : **AT BE CH DE FR GB IT LU NL SE**

(56) Entgegenhaltungen : **US A 4 097 489**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Skuballa, Werner, Dr.**
**Olwenstrasse 25**
**D-1000 Berlin 28 (DE)**
Erfinder : **Radüchel, Bernd, Dr.**
**Gollanczstrasse 132**
**D-1000 Berlin 28 (DE)**
Erfinder : **Vorbrüggen, Helmut, Prof.**
**Wilkestrasse 7**
**D-1000 Berlin 27 (DE)**
Erfinder : **Mannesmann, Gerda, Dr.**
**Hagenplatz 3e**
**D-1000 Berlin 33 (DE)**
Erfinder : **Losert, Wolfgang, Prof.**
**Niedstrasse 12**
**D-1000 Berlin 41 (DE)**
Erfinder : **Casals, Jorge, Dr.**
**Wichernstrasse 20**
**D-1000 Berlin 33 (DE)**

**EP 0 011 591 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Neue Prostanderivate, ihre Herstellung und sie enthaltende Arzneimittel

Die Erfindung betrifft neue Prostacyclin-Derivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefäße (Science *196*, 1072) und ist daher als Mittel zur Blutdrucksenkung anzusehen. $PGI_2$ besitzt jedoch nicht die für ein Arzneimittel notwendige Stabilität. So beträgt die Halbwertszeit von $PGI_2$ bei physiologischen pH-Werten und bei Raumtemperatur nur wenige Minuten.

In dem US-Patent 4,097,489 werden 9-Desoxyprostacycline beschrieben, in denen der 9-Sauerstoff durch Stickstoff ersetzt worden ist.

Es wurde gefunden, daß der Ersatz des 9-Äthersauerstoffatoms im Prostacyclin durch eine Methylengruppe zu einer Stabilisierung des Prostacyclins führt, wobei das pharmakologische Wirkungsspektrum erhalten bleibt und die Wirkungsdauer deutlich verlängert wird.

Die erfindungsgemäßen Verbindungen wirken blutdrucksenkend und bronchodilatatorisch. Sie sind außerdem zur Inhibierung der Thrombozyten-aggregation geeignet.

Die Erfindung betrifft Prostanderivate der allgemeinen Formel I

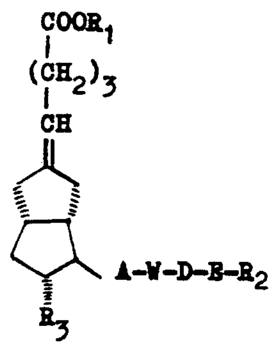

(I)

worin

$R_1$ Wasserstoff, Alkyl mit 1-10 C-Atomen, das durch Halogen, Phenyl, Dimethylamino, Diäthylamino, Methoxy oder Äthoxy substituiert sein kann, Cycloalkyl mit 5 oder 6 C-Atomen, Phenyl, das in 3- oder 4-Stellung durch Halogen, $C_1$-$C_4$-Alkoxy, Hydroxy oder Trifluormethyl substituiert sein kann, oder einen 5- oder 6-gliedrigen heterocyclischen Rest mit mindestens einem Stickstoff-, Sauerstoff- oder Schwefelatom,

A eine $—CH_2—CH_2—$, trans—CH = CH— oder —C ≡ C—Gruppe,

W eine freie oder an der hydroxygruppe funktionell abgewandelte Hydroxymethylengruppe oder eine freie oder an der Hydroxygruppe funktionell abgewandelte

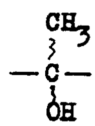 Gruppe

wobei die OH-Gruppe α- oder β-ständig ist,

D und E gemeinsam eine direkte Bindung oder

D eine geradkettige oder verzweigte gesättigte Alkylengruppe mit 1-10 C-Atomen oder eine ungesättigte Alkylengruppe mit bis zu 10 C-Atomen, die gegebenenfalls durch Fluoratome substituiert sind,

E ein Sauerstoffatom oder eine $—C_3C—$Bindung oder eine direkte Bindung,

$R_2$ eine gerad- oder verzweigtkettige, gesättigte oder ungesättigte Alkylgruppe mit 1-7 C-Atomen, eine Cycloalkylgruppe mit 5 oder 6 C-Atomen, eine durch Halogen, Phenyl, $C_1$-$C_4$-Alkyl, Chlormethyl, Fluormethyl, Trifluormethyl, Hydroxy oder Carboxyl substituierte Phenylgruppe oder einen 5- oder 6-gleidrigen heterocyclischen Rest mit mindestens einem Stickstoff-, Sauerstoff- oder Schwefelatom,

$R_3$ eine freie oder funktionell abgewandelte Hydroxygruppe,

und falls $R_1$ die Bedeutung eines Wasserstoffatoms hat, deren Salze mit physiologisch verträglichen Basen bedeuten.

Als Alkylgruppe $R_1$ sind gerade oder verzweigte Alkylgruppe mit 1-10 C-Atomen zu betrachten, wie beispielsweise Methyl, Äthyl, Propyl, Butyl, Isobutyl, tert.-Butyl, Pentyl, Neopentyl, Heptyl, Hexyl, Decyl. Die Alkylgruppen $R_1$ können gegebenenfalls 1 bis mehrfach substituiert sein durch Halogenatome. Bevorzugt sind solche Alkylgruppen, die einfach substituiert sind.

Als Substituenten seien beispielsweise genannt Fluor-, Chlor- oder Bromatome. Als bevorzugte

Alkylgruppen $R_1$ sind solche mit 1-4 C-Atomen, wie z.B. Methyl, Äthyl, Propyl, Dimethylaminopropyl, Isobutyl, Butyl zu nennen.

In den Phenylgruppen $R_1$ sind die Substituenten, in 3- und 4-Stellung am Phenylring bevorzugt Fluor, Chlor oder Trifluormethyl oder in 4-Stellung Hydroxy.

Die Cycloalkylgruppe $R_1$ mit 5 oder 6 Kohlenstoffatomen ist Cyclopentyl oder Cyclohexyl. Als heterocyclische Reste $R_1$ kommen beispielsweise 2-Furyl, 2-Thienyl, 2-Pyridyl, 3-Pyridyl und 4-Pyridyl in Frage.

Die Hydroxygruppen $R_3$ und in W können funktionell abgewandelt sein, beispielsweise durch Verätherung oder Veresterung, wobei die freien oder abgewandelten Hydroxygruppen in W α- oder β-ständig sind, wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butylsilyl- und Tribenzyl-silylrest. Als Acylreste kommen beispielsweise in Frage : Acetyl, Propionyl, Butyryl, Benzoyl.

Als Alkylgruppen $R_2$ seien beispielsweise Methyl, Äthyl, Propyl, Butyl, Isobutyl, tert.-Butyl, Pentyl, Hexyl, Heptyl, Butenyl, Isobutenyl, Propenyl, Pentenyl und Hexenyl-genannt.

Die Cycloalkylgruppe $R_2$ mit 5 oder 6 Kohlenstoffatomen ist Cyclopentyl oder Cyclohexyl.

Die Phenyl gruppe $R_2$ kann durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, oder Hydroxygruppe substituiert sein. Bevorzugt ist die Substitution in 3- und 4-Stellung am Phenylring zum Beispiel durch Fluor, Chlor oder Trifluormethyl oder in 4-Stellung durch Hydroxy. Als heterocyclische Gruppen $R_2$ seien beispielsweise 2-Furyl, 2-Thienyl, 2-Pyridyl, 3-Pyridyl, 4-Pyridyl, Oxazolyl, Thiazolyl, Perimidinyl, Pyridazinyl und Pyrazinyl genannt.

Als Alkylengruppe D kommen vorzugsweise gesättigte Alkylengruppen, insbesondere solche mit 1-5 C-Atomen, in Frage, die gegebenenfalls durch Fluoratome substituiert sein können. Beispielsweise seien genannt : Methylen, Fluormethylen, Difluormethylen, Äthylen, 1.2-Propylen, Äthyläthylen, Trimethylen, Tetramethylen, Pentamethylen, 1.1-Difluor-äthylen, 1-Fluor-äthylen, 1-Methyl-tetramethylen, 1-Methyltrimethylen, 1-Methylen-äthylen, 1-Methylen-tetramethylen. Zur Salzbildung mit den freien Säuren ($R_1$ = H) sind anorganische und organische Basen geeignet, wir sie dem Fachmann zur Bildung physiologisch verträglicher Salze bekannt sind. Beispielsweise seien genannt : Alkalihydroxide, wie Natrium- und Kaliumhydroxid, Erdalkalihydroxide, wie Calciumhydroxid, Ammoniak, Amine, wie Äthanolamin, Diäthannolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostanderivate der allgemeinen Formel I, dadurch gekennzeichnet, daß man in an sich bekannter Weise eine Verbindung der allgemeinen Formel II,

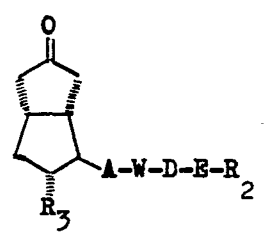

(II)

worin $R_2$, $R_3$, A, W, D und E die vorstehendangegebenen Bedeutungen aufweisen, gegebenenfalls nach Schutz anwesender freier Hydroxygruppen mit einem Wittig-Reagenz der allgemeinen Formel III

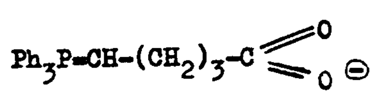

(III)

worin Ph eine Phenylgruppe bedeutet, umsetzt und gegebenenfalls anschließend in beliebiger Reihenfolge Isomere trennt und/oder geschützte Hydroxygruppen freisetzt und/oder freie Hydroxygruppen verestert oder veräthert und/oder eine freie Carboxylgruppe verestert und/oder eine veresterte Carboxylgruppe verseift oder eine Carboxylgruppe mit eine physiologisch verträglichen Base in ein Salz überführt.

Die Umsetzung der Verbindung der allgemeinen Formel II mit dem Wittig-Reagenz der allgemeinen Formel III, das man aus dem entsprechenden Phosphoniumsalz mit Methansulfinylmethylnatrium oder Methansulfinylmethylkalium oder Kalium tert.-butylat in Dimethylsulfoxid herstellt, wird bei Temperaturen von 0-100 °C, vorzugsweise bei 20-80 °C, in einem aprotischen Lösungsmittel, vorzugsweise Dimethylsulfoxid oder Dimethylformamid, vorgenommen. Die Trennung der dabei erhaltenen Z- und E-konfigurierten Olefine erfolgt auf übliche Art, beispielsweise durch Säulen- oder Schichtchromatographie.

Die Verseifung der Prostaglandinester wird nach den dem Fachmann bekannten Methoden

durchgeführt, beispielsweise mit basischen Katalysatoren. Die Einführung der Estergruppe, bei welcher $R_1$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermisch. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. *8,* Seiten 389-394 (1954)].

Die Einführung der Estergruppe $R_1$, bei welcher $R_1$ eine wie im Anspruch 1 angegeben substituierte oder unsubstituierte Phenylgruppe darstellt, erfolgt nach den dem Fachmann bekannten Methoden. Beispielsweise werden die Carboxyverbindungen mit den entsprechenden Phenylhydroxyverbindungen mit Dicyclohexylcarbodiimid in Gegenwart einer geeigneten Base, beispielsweise Pyridin oder Triäthylamin, in einem interten Lösungsmittel umgesetzt. Als Lösungsmittel kommen Methylenchlorid, Äthylenchlorid, Chloroform, Essigester, Tetrahydrofuran, vorzugsweise Chloroform, in Frage. Die Reaktion wird bei Temperaturen zwischen $-30\,°C$ und $+50\,°C$, vorzugsweise bei $+10\,°C$, durchgeführt.

Die Prostaglandin-Derivate der allgemeinen Formel I mit $R_1$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren mit Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmlann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluol-sulfonsäure, umgesetzt. Das Dihydropyran wird im Überschuß angewandt, vorzugsweise in der 2- bis 10-fachen Menge des theoretischen Bedarfs. Die Umsetzung ist normalerweise bei 0°-30 °C nach 15-30 minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer organischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wir Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei $-10\,°C$ bis 70 °C, vorzugsweise bei 25 °C.

Das als Ausgangsmaterial für das vorstehend beschriebene Verfahren verwendete Keton der allgemeinen Formel II aknn hergestellt werden, indem man einen Alkohol der Formel IV

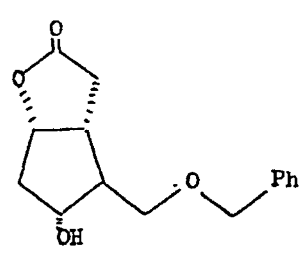

(IV)

(E.J. Corey et al., J. Amer. Chem. Soc. *93,* 1490 (1971)) mit Dihydropyran in Anwesenheit katalytischer Mengen p-Toluolsulfonsäure in den Tetrahydropyranyläther V überführt.

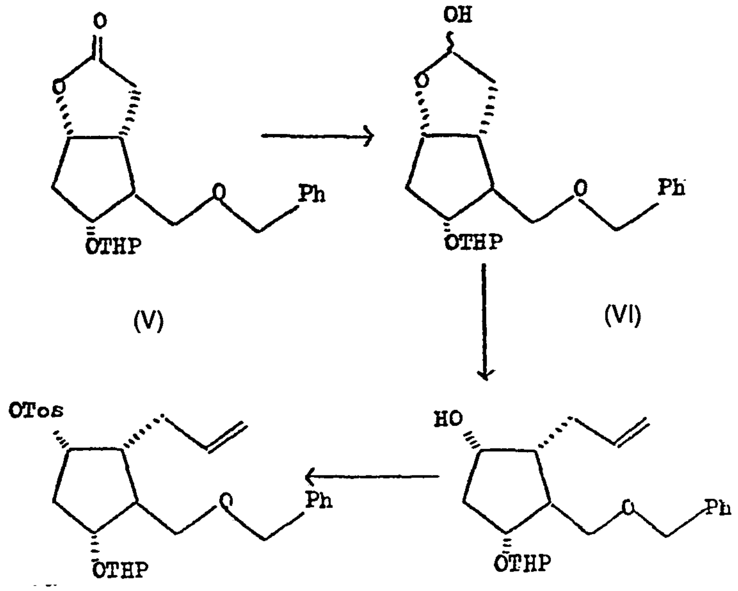

(V) (VI)

(VIII) (VII)

Das Lacton V wird mit Diisobutylaluminiumhydrid bei − 70 °C zum Lactol VI reduziert, das durch Wittigreaktion mit Triphenylphosphoniummethylen in das Olefin VII überführt wird. Nach Überführung in das Tosylat mit p-Toluolsulfonylchlorid in Gegenwart von Pyridin erhält man durch Umsetzung mit Kaliumnitrit in Dimethylsulfoxyd den 9β-konfigurierten Alkohol IX, der mit p-Toluolsulfonylchlorid in Gegenwart von Pyridin in das Tosylat X überführt wird. Umsetzung mit Malonsäurediäthylester in Gegenwart von Kalium-tert-butylat liefert den Diester XI, der durch Decarbalkoxylierung mit Natriumcyanid in Dimethylsulfoxid in den Ester XII umgewandelt wird.

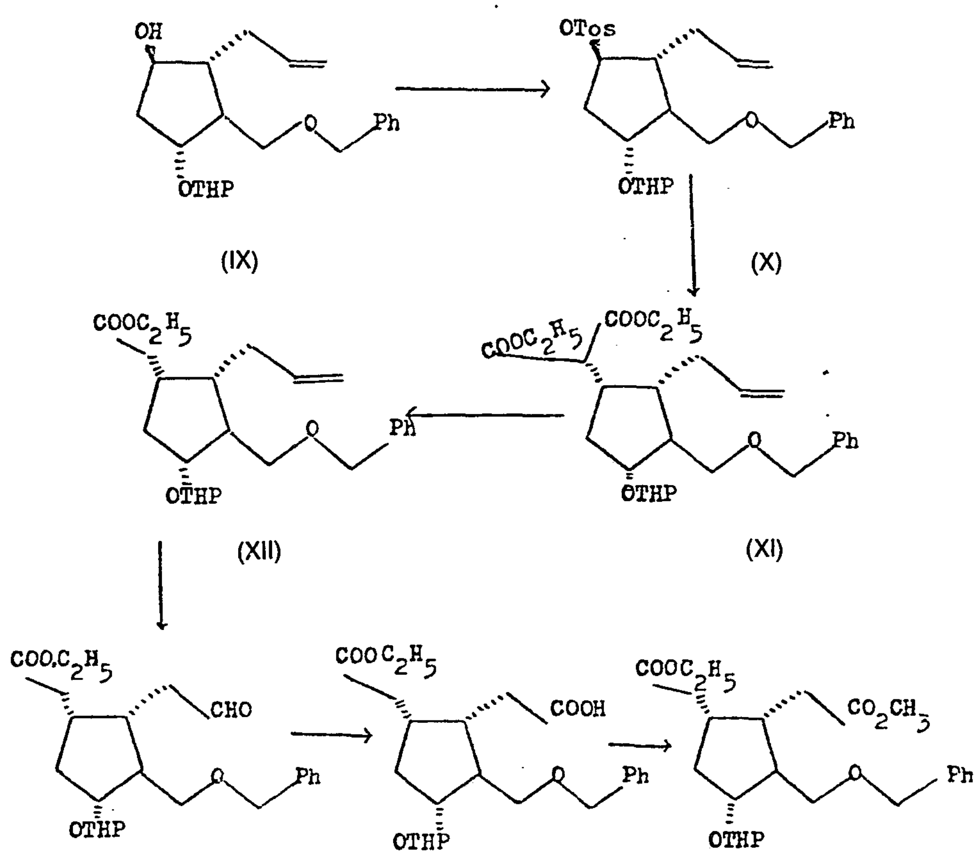

(IX) (X)

(XII) (XI)

XIII XIV XV

Oxidative Spaltung der Doppelbindung in der Verbindung XII mit Natriumperjodat in Gegenwart katalytischer Mengen Osmiumtetroxid führt zum Aldehyd XIII, der mit Jones-Reagenz zur Säure XIV oxidiert wird, die anschließend mit Diazomethan zur Verbindung XV verestert wird. Durch Dieckmannkondensation von XV mit Kalium-tert-butylat in Tetrahydrofuran erhält man ein Isomerengemisch der Ketocarbonsäureester XVI und XVII, das mittels einer Decarbalkoxylierung mit 1,4-Diazabicyclo[2.2.2]octan in Xylol in das Keton XVIII als einziges Reaktionsprodukt überführt wird.

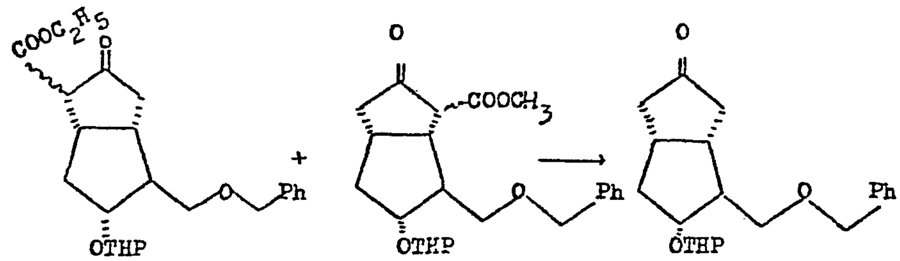

XVI XVII XVIII

Die Abspaltung der Tetrahydropyranylätherschutzgruppe liefert den Alkohol XIX, der mit Benzoylchlorid in Gegenwart von Pyridin zum Ester XX verestert wird.

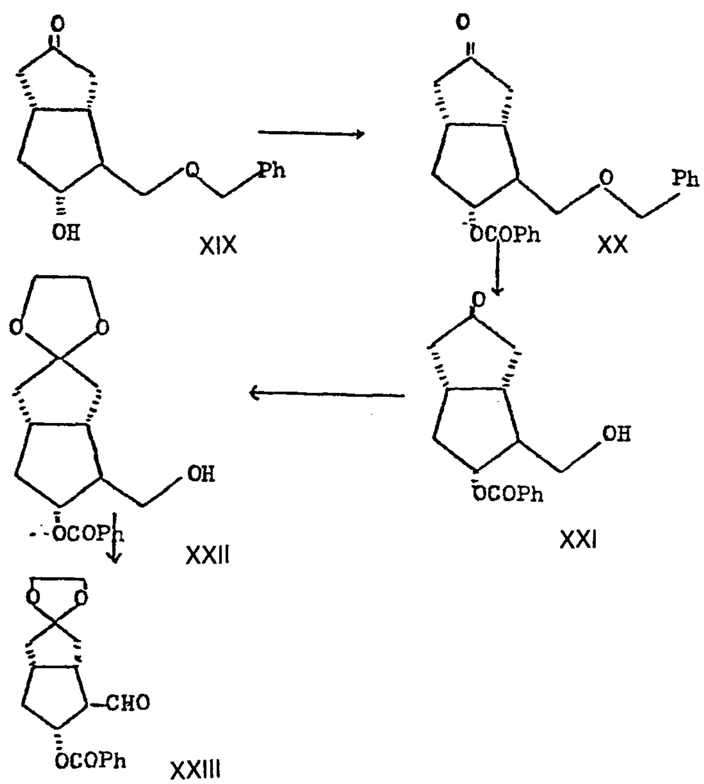

XIX XX XXI XXII XXIII

Hydrogenolytische Spaltung des Benzyläthers in Gegenwart katalytischer Mengen Säure ergibt den Alkohol XXI, der nach Ketalisierung zur Verbindung XXII mit Collins-Reagenz zum Aldehyd XXIII oxidiert wird.

Dieser Aldehyd wird mit einem Phosphonat der allgemeinen Formel XXIV

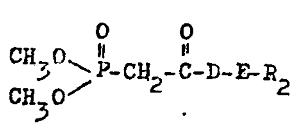

XXIV

worin D, E und $R_2$ die obenangegebene Bedeutung aufweisen, in einer Olefinierungsreaktion zu einem Keton der allgemeinen Formel XXV umgesetzt.

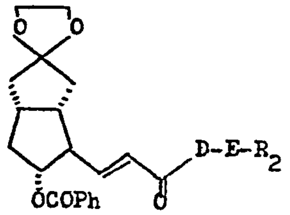

XXV

Nach Reduktion der 15-Ketogruppe mit Zinkborhydrid oder Natriumhydrid oder Umsetzung mit Alkylmagnesiumbromid oder Alkyllithium und Epimerentrennung erhält man die 15α-Alkohole XXVI (PG-Numerierung).

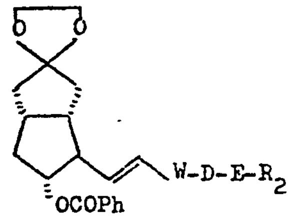

XXVI

Nach Verseifung der Estergruppe beispielsweise mit Kaliumcarbonat in Methanol und Ketalpaltung mit wäßriger Essigsäure erhält man das Keton der allgemeinen Formel XXVII,

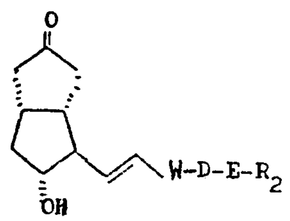

XXVII

welches gegebenenfalls nach funktioneller Abwandlung der freien Hydroxygruppen, beispielsweise durch Verätherung mit Dihydropyran oder gegebenenfalls Hydrierung der Doppelbindung, in die Verbindung der allgemeinen Formel II umgewandelt wird.

Die Verbindungen dieser Erfindung wirken blutdrucksenkend und bronchodilatorisch. Sie sind weiterhin geeignet zur Hemmung der Thrombozyten-Aggregation. Folglich stellen die neuen Postacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Sepzifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z.B. am Meerschweincheniteum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z.B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken ; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut ; antiallergische Eigenschaften, Snekung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Prostaglandinanaloga antiprolifertive Eigenschaften.

Die Dosis der Verbindungen ist 1-1 500 µg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeingIußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Bezugsbeispiel 1

a) (1S, 5R, 6S, 7R)-6-Benzyloxymethyl-7-(tetrahydropyran-2-yl-oxy)-2-oxabicyclo[3.3.0]octan-3-on

Eine Lösung aus 14,5 g (1S, 5R, 6S, 7R)-6-benzyloxymethyl-7-hydroxy-2-oxabicyclo[3.3.0]octan-3-on, 6 ml Dihydropyran und 40 mg p-Toluolsulfonsäure in 300 ml abs. Methylenchlorid, rührt man 1 Stunde bei 5 °C. Anschließend verdünnt man mit Äther, schüttelt mit 4 %iger Natriumbikarbonatlösung, wäscht mit Wasser neutral, trocknet über Magnesiumsulfat und dampft im Vakuum ein. Man erhält 21 g des Tetrahydropyranyläthers als farbloses Öl.

IR : 2 950, 2 860, 1 770, 1 458/cm.

b) (1SR, 3RS, 5R, 6S, 7R)-6-Benzyloxymethyl-3-hydroxy-7-(Tetrahydropyran-2-yloxy)-2-oxabicyclo[3.3.0.]octan

Zu einer Lösung von 21 g des nach Bezugsbeispiel la hergestellten Produkts in 940 ml abs. Toluol tropft man bei − 70° unter Argon 160 ml 1,2M Lösung von Diisobutylaluminiumhydrid in Toluol. Man rührt 30 Minuten bei − 70 °C, versetzt tropfenweise mit 5 ml Isopropanol, läßt auf − 10° erwärmen, versetzt mit 70 ml Wasser, rührt 2 Stunden bei Raumtemperatur, filtriert und dampft im Vakuum ein. Dabei erhält man 20 g des Lactols als farbloses Öl.

IR : 3 600, 3 400 (br.), 2 950, 2 860, 1 457/cm.

1c) (1S, 2R, 3S, 4R)-3-Benzyloxymethyl-2-(prop-2-en-1-yl)-4-(tetrahydropyran-2-yloxy)-cyclopentanol

Zu einer Lösung von 28,6 g Methyltriphenylphosphoniumbromid in 75 ml abs. Dimethylsulfoxid (DMSO) tropft man bei 15 °C unter Argon 77 ml einer 1,04 M Lösung von Methylsufinylmethylnatrium in DMSO und rührt eine Stunde bei Raumtemperatur. Anschließend fügt man eine Lösung von 7 g des nach Bezugsbeispiel 1b hergestellten Lactols in 50 ml abs. DMSO zu und rührt 3 Stunden bei Raumtemperatur. Man rührt im 500 ml Eiswasser ein, extrahiert viermal mit je 200 ml einer Mischung aus Pentan/Äther (1 + 1), wäscht den organischen Extrakt mit Wasser neutral, trocknet über Magensiumsulfat und dampft im Vakuum ein.

Der Rücksfand wird durch Chromatographie an Kieselgel mit Pentan/Äther-Gradienten gereinigt. Man erhält 6,1 g des Cyclopentanols als farbloses Öl.

IR : 3 500, 2 940, 2 855, 920, 968, 998/cm.

1d) (1S, 2R, 3S, 4R)-3-Benzyloxymethyl-2-(prop-2-en-1-yl)-1-tosyloxy-4-(tetrahydropyran-2-yloxy)-cyclopentan

Zu einer Lösung von 12,5 g des nach Bezugsbeispiel 1c hergestellten Alkohols in 36 ml Pyridin fügt man 13,6 g p-Toluolsulfonylchlorid und rührt 2 Tage bei Raumtemperatur unter Argon. Anschließend versetzt man mit 6 ml Eiswasser, rührt 2 Stunden bei Raumtemperatur, verdünnt mit 0,6 l Äther und schüttelt nacheinander mit Wasser, eiskalter 4 %iger Schwefelsäure, Wasser, 5 %iger $NaHCO_3$-Lösung, 3x mit Wasser. Man trocknet über Magnesiumsulfat und dampft im Vakuum ein. Man erhält 17,3 g des Tosylats als farbloses Öl.

IR : 2 950, 2 863, 1 600, 1 365, 1 175, 903/cm.

1e) (1R, 2R, 3S, 4R)-3-benzyloxymethyl-2-(prop-2-en-1-yl)-4-tetrahydropyran-2-yloxy)-cyclopentanol

Zu einer Lösung von 17 g des nach 1d hergestellten Tosylats in 500 ml DMSO fügt man 5 lg Kaliumnitrit und rührt 2,5 Stunden bei 65 °C unter Argon. Anschließend gießt man auf eine 20 %ige

Natriumchloridlösung, extrahiert 5x mit je 250 ml einer Mischung aus Pentan/Äther (1 + 1), wäscht die organische Phase dreimal mit je 100 ml Sole, trocknet über Magnesiumsulfat und dampft im Vakuum ein. Nach Chromatographie des Rückstandes an Kieselgel erhält man mit Äther/Pentan (3 + 2) 7,8 g des invertierten Alkohols als farbloses Öl.

IR : 3 600, 3 450, 2 950, 2 864, 1 000, 974, 918/cm.

1f) (1R, 2R, 3S, 4R)-3-benzyloxymethyl-2-(prop-2-en-1-yl)-4-(tetrahydropyran-2-yloxy)-1-toxyloxy-cyclopentan

In Analogie zu Bezugsbeispiel 1d erhält man aus 6,8 g des nach le hergestellten Alkohols 9,5 g des Tosylats als farbloses Öl.

IR : 2 950, 2 863, 1 645, 1 602, 1 375, 1 177, 975, 910/cm.

1g) (1S, 2S, 3S, 4R)-1-(Bis-äthoxycarbonyl)-methyl-3-benzyloxymethyl-2-(prop-2-en-1-yl)-4-(tetrahydropyran-2-yloxy-cyclopentan

Zu einer Lösung von 30,2 g Malonsäurediäthylester in 125 ml t-Butanol fügt man 10,6 g Kalium-t-butylat und rührt 1 Stunde bei 60-80 °C, anschließend fügt man eine Lösung von 9,5 g des nach 1f hergestellten Tosylats in 45 ml t-Butanol zu und rührt 26 Stunden unter Rückfluß und Argon. Anschließend verdünnt man mit Ähter, wäscht mit Wasser neutral, trocknet über Magnesiumsulfat und dampft im Vakuum ein. Den Rückstand befreite man durch eine Kieselrohrdestillation bei 0,1 Torr und 60-80 °C von flüchtigen Anteilen. Nach Reinigung durch Chromatographie an Kieselgel mit Pentan/Essigester (4 + 1) erhält man 5,5 g des obigen Diesters als farbloses Öl.

IR : 2 950, 2 860, 1 750, 1 730, 1 642, 973, 915/cm.

1h) (1R, 2S, 3S, 4R)-Äthoxycarbonylmethyl-3-benzyloxymethyl-2-(prop-2-en-yl)-4-(tetrahydropyran-2-yloxy)-cyclopentan

4,60 g des nach Bezugsbeispiel hergestellten Diesters, 1,06 g Natriumcyanid in 30 ml DMSO rührt man 20 Stunden bei 150 °C unter Argon, anschließend verdünnt man mit 300 ml einer Mischung aus Äther/Pentan (1 + 1), schüttelt dreimal mit je 50 ml Wasser und trocknet über Magnesiumsulfat und dampft im Vakuum ein. Nach Reinigung durch Chromatographie an Kieselgel erhält man mit Pentan/Essigester (9 + 1) 2,80 g des Monoesters als farbloses Öl.

IR : 2 950, 2 860, 1 730, 1 643, 973, 916/cm.

1i) (1R, 2S, 3S, 4R)-1-Äthoxycarbonylmethyl-3-benzyloxy-methyl-2-formylmethyl-4-(tetrahydropyran-2-yloxy)-cyclopentan

Zu einer Lösung von 2,5 g des nach Bezugsbeispiels 1h) hergestellten Esters in 31 ml Tetrahydrofuran und 7,8 ml Wasser fügt man bei 25 °C eine Lösung von 16 mg Osniumtetroxid in 2 ml Tetrahydrofuran. Anschließend versetzt man innerhalb von 45 Minuten portionsweise mit 3,2 g Natriumperjodat, rührt 30 Minuten bei 25 °C, filtriert, verdünnt mit Äther, schüttelt einmal mit verd. Natriumhydrogensulfitlösung und wäscht mit Wasser neutral. Man trocknet über Magnesiumsulfat und dampft im Vakuum ein. Dabei erhält man 2,48 g des Aldehyds als hellgelbes Öl.

IR : 2 950, 2 860, 2 730, 1 725, 970/cm.

1j) (1R, 2S, 3S, 4R)-1-Äthoxycarbonylmethyl-3-benzyloxy-methyl-2-carboxymethyl-4-(tetrahydropyran-2-yloxy)-cyclopentan

Zu einer Lösung von 2,5 g des nach 1i hergestellten Aldehyds in 60 ml Aceton tropft man bei 5 °C 2,5 ml Jones-Reagenz und rührt 45 Minuten bei 5 °C. Anschließend versetzt man überschüßiges Reagenz durch tropfenweise Zugabe on Isopropanol, rührt 5 Minuten, verdünnt mit Äther und wäscht man Wasser neutral, trocknet über Magnesiumsulfat, dampft im Vakuum ein und reinigt den Rückstand durch Säulenchromatographie an Kieselgel. Mit Pentan/Essigester (3 + 2) erhält man 1,9 g der Carbonsäure als farbloses Öl.

IR : 3 500 (breit), 2 950, 2 860, 1 725, 970/cm.

1k) (1R, 2S, 3S, 4R)-1-Äthoxycarbonylmethyl-3-benzyloxy-methyl-2-methoxycarbonyl-4-(tetrahydropyran-2-yloxy)-cyclopentan

Zu einer Lösung aus 1,3 g der nach Bezugsbeispiel 1j hergestellten Säure in 30 ml Methylenchlorid tropft man bei Eisbadtemperatur 7 ml einer ätherischen Diazomethanlösung, rührt 3 Minuten und dampft im Vakuum ein.

Dabei erhält man 1,3 g des Methylesters als farbloses Öl.

IR : 2 958, 2 860, 1 731, 970/cm.

1l) (1R, 5S, 6S, 7R)-6-Benzyloxymethyl-7-(tetrahydropyran-2-yloxy)-bicyclo[3.3.0]octan-3-on

Eine Mischung aus 1,3 g des nach Bezugsbeispiel 1k hergestellten Diesters und 3 g Kalium-tert. -butylat in 60 ml Tetrahydrofuran rührt man 3 Stunden bei Raumtemperatur unter Argon. Anschließend säuert man mit 10 %iger Zitronensäurelösung auf pH 5 an, verdünnt mit Äther und wäscht mit Wasser neutral. Man trocknet über Magnesiumsulfat und dampft im Vakuum ein. Dabei erhält man 1,2 g eines Gemisches aus dem stereoisomeren ß-Ketoesters.

Zur Decarbalkoxylierung löst man das Rohprodukt in 24 ml Xylol, setzt 2,4 g 1,4-Diazabicyclo/2.2.2./ octan zu und rührt 4 Stunden bei 160° Badtemperatur unter Argon. Anschließend verdünnt man mit Äther, schüttelt nacheinander mit Wasser, eiskalter 3 %iger Schwefelsäure, Natriumbikarbonatlösung und Wasser, trocknet über Magnesiumsulfat und dampft im Vakuum ein. Man erhält 980 mg des Ketons als hellgelbel Öl.

IR : 2 935, 2 860, 1 735, 970/cm.

1m) (1R, 5S, 6S, 7R)-6-Benzyloxymethyl-7-hydroxy-bicyclo[3.3.0]octan-3-on

0,9 g des nach Bezugsbeispiel 1l hergestellten Ketons rührt man 3 Stunden bei 45 °C mit 17 ml einer Mischung aus Essigsäure/Wasser/Tetrahydrofuran (65/35/10) und dampft anschließend im Vakuum ein. Den Rückstand reinigt man durch Chromatographie an Kieselgel. Mit Pentan/Essigester (3 + 2) erhält man 0,68 g des Alkohols als farbloses Öl.

IR : 3 540, 2 935, 2 860, 1 739, 1 095/cm.

1n) (1R, 5S, 6S, 7R)-7-Benzoyloxy-6-benzyloxymethyl-bicyclo[3.3.0]octan-3-on

Zu einer Lösung von 0,55 g des nach Bezugsbeispiel 1m hergestellten Alkohols im 4 ml Pyridin gibt man 0,5 ml Benzoylchlorid, rührt 4 Stunden bei 25 °C, versetzt mit 0,4 ml Wasser, rührt 2 Stunden, verdünnt mit Äther, schüttelt nacheinander mit Wasser, 5 %iger Schwefelsäure, Wasser, 4 %iger Natriumbikarbonatlösung und dreimal mit Wasser. Nach Trocknen über Magnesiumsulfat dampft man im Vakuum ein und erhält 720 mg des Benzoats als farbloses Öl.

IR : 2 945, 2 860, 1 739, 1 713, 1 602, 1 588, 1 276/cm.

1o) (1R, 5S, 6S, 7R)-7-Benzoyloxy-6-hydroxymethyl-bicyclo[3.3.0]octan-3-on

Eine Lösung von 680 mg des nach Bezugsbeispiel 1n hergestellten Benzoats in 10 ml Essigester und 0,5 ml Eisessig wird mit 120 mg Palladium auf Kohle (10 %ig) versetzt und 8 Stunden unter einer Wasserstoffatmosphäre geschüttelt. Nach Filtrieren und Eindampfen der Lösung im Vakuum erhält man 600 mg eines öligen Rohprodukts, das durch Chromatographie an Kieselgel mit Pentan/Essigester (1 + 1) gereinigt wird und 395 mg des sauberen Alkohols als farbloses Öl.

IR : 3 500, 2 945, 1 739, 1 712, 1 602, 1 588, 1 278/cm.

1p) (1R, 5S, 6S, 7R)-3,3-Äthylendioxy-7-benzoyloxy-6-hydroxymethyl-bicyclo[3.3.0]octan

320 mg des nach Beispiel 1o hergestellten Alkohols, 0,5 ml Äthylenglycol, 4 mg p-Toluolsulfonsäure und 10 ml Benzol rührt man 1,5 Stunden mit einem Wasserabscheider bei Rückflußtemperatur. Man kühlt ab, verdünnt mit Äther, schüttelt einmal mit 4 %iger Natriumbikarbonatlösung, wäscht mit Wasser neutral, trocknet über Magnesiumsulfat und dampft im Vakuum ein. Dabei erhält man 390 mg des Ketals als farbloses Öl.

IR : 3 500, 2 945, 2 882, 1 708, 1 604, 1 588, 1 280, 948/cm.

1q) (1R, 5S, 6R, 7R)-3,3-Äthylendioxy-7-benzoyloxy-6-formyl-bicyclo[3.3.0]octan

Zu einer Lösung von 5,4 g Collins-Reagenz in 63 ml abs. Methylenchlorid tropft man unter Rühren bei 5 °C eine Lösung von 1,03 g des nach Bezugsbeispiel 1p hergestellten Ketals in 32 ml abs. Methylenchlorid und rührt 20 Minuten bei 5 °C. Anschließend verdünnt man mit Äther, schüttelt dreimal mit Natriumbikarbonatlösund, dreimal mit Sole, trocknet über Magnesiumsulfat und dampft im Vakuum bei 25 °C ein. Man erhält 840 mg des Aldehyds als gelbes Öl.

IR : 2 960, 2 730, 1 720, 1 603, 1 588, 1 275, 948/cm.

Beispiel 1

5-{(E)-(1S, 5S, 6R, 7R)-7-Hydroxy-6-[(E)-3α-hydroxyl-1-octenyl]-bicyclo[3.3.0]octan-3-yliden}-pentansäure

Zu einer Lösung von 3,04 g 4-Carboxylbutyltriphenylphosphoniumbromid in 6 ml trockenem Dimethylsulfoxid (DMSO) tropft man bei 15 °C unter Argon 12 ml einer 1,04 molaren Lösung von

Methylsulfinylmethylnatrium in DMSO und rührt 30 Minuten bei Raumtemperatur. Zur roten Ylenlösung tropft man eine Lösung von 495 mg (1R, 5S, 6R, 7R)-7-(Tetrahydropyran-2-yloxy)-6-[(E)-(3S)-3-(tetrahydropyran-2-yloxy)-1-octenyl]-bicyclo[3.3.0]-octan-3-on in 3 ml abs. DMSO und rührt 2 Std. bei 45 °C. Das Reaktionsgemisch wird auf Eiswasser gegossen, mit 10 %iger Zitronensäurelösung auf pH 4-5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Pentan (3 + 2) 462 mg des Olefinierungsprodukts, welches man zur Abspaltung der Schutzgruppen mit 15 ml einer Mischung aus Essigsäure/Wasser/Tetrahydrofuran (65/35/10) 20 Stunden bei 25 °C rührt. Man dampft im Vakuum ein und chromatographiert den Rückstand an Kieselgel. Mit Methylenchlorid/Isopropanol (95 + 5) erhält man zunächst 65 mg 5-{(Z)-(1S, 5S, 6R, 7R)-7-Hydroxy-6-[(E)-(3S)-3-hydroxy-1-octenyl]-bicyclo[3.3.0]octan-3-yliden}-pentansäure (Schmelzpunkt 95 °C) sowie als polarere Komponente 103 mg der Titelverbindung als farbloses Öl.

IR : 3 600, 3 450 (breit), 2 940, 2 865, 1 712, 1 604, 975/cm.

Das Ausgangsmaterial für die obige Titelverbindung wird wie folgt hergestellt :

1a) (1R, 5S, 6R, 7R)-3,3-Äthylendioxy-7-benzoyloxy-6-[(E)-3-oxo-1-octenyl]-bicyclo[3.3.0]octan

Zu einer Suspension von 126 mg Natriumhydrid (55 %ige Suspension in Öl) in 11 ml Dimethoxyäthan (abs.) (DME) tropft man bei Raumtemperatur eine Lösung von 664 mg 2-Oxo-heptylphosphonsäuredimethylester in 5,5 ml DME (abs.), rührt 10 Minuten, fügt 121 mg Lithiumchlorid zu und rührt 2 Std. bei Raumtemperatur unter Argon. Anschließend versetzt man bei −20 °C mit einer Lösung von 755 mg des nach Bezugsbeispiel 1q hergestellten Aldehyds in 11 ml DME (abs.) und rührt 2,5 Std. bei Raumtemperatur unter Argon. Anschließend gießt man das Reakltionsgemisch auf 120 ml gesättigte Ammoniumchloridlösung, extrahiert dreimal mit Äther, wäscht den organischen Extrakt mit Wasser neutral, trocknet über Magnesiumsulfat und dampft im Vakuum ein. Den Rückstand reinigt man durch Chromatographie an Kieselgel. Mit Äther/Pentan (1 + 1) erhält man 795 mg der Titelverbindung als farbloses Öl.

IR : 2 940, 2 862, 1 715, 1 670, 1 628, 1 275, 979, 948/cm.

1b) (1R, 5S, 6R, 7R)-3,3-Äthylendioxy-7-benzoyloxy-6-[(E)-3α-hydroxy-1-octenyl]-bicyclo[3.3.0]octan

Zu einer Lösung von 790 mg des nach Beispiel 1a hergestellten Ketons in 24 ml Methanol fügt man bei −40 °C portionsweise 420 mg Natriumborhydrid und rührt 1 Stunde bei −40 °C unter Argon. Anschließend verdünnt man mit Äther, wäscht mit Wasser neutral, trocknet über Magnesiumsulfat und dampft im Vakuum ein. Durch Säulenchromatographie an Kieselgel mit Äther/Pentan (7 + 3) erhält man zunächst 245 mg der Titelverbindung als farbloses Öl. Als polarere Komponente erhält man 152 mg (1R, 5S, 6R, 7R)-3,3-Athylendioxy-7-benzoyloxy-6-[(E)-(3R)-3-hydroxy-1-octenyl]-bicyclo[3.3.0]octan.

IR : 3 610, 3 400 (breit), 2 940, 1 715, 1 604, 1 588, 1 279, 971, 948/cm.

1c) (1R, 5S, 6R, 7R)-3,3-Äthylendioxy-7-hydroxy-6-[(E)-3α-hydroxy-1-octenyl]-bicyclo[3.3.0]octan

Eine Mischung aus 500 mg des nach Beispiel 1b hergestellten α-Alkohols und 333 mg Kaliumcarbonat (wasserfrei) in 35 ml Methanol rührt man 16 Stunden bei Raumtemperatur unter Argon. Anschließend engt man im Vakuum ein, verdünnt mit Äther und wäscht mit Sole neutral. Man trocknet über Magnesiumsulfat und dampft im Vakuum. Dabei erhält man 495 mg der Titelverbindung als farbloses Öl (Rohprodukt).

IR : 3 600, 3 450 (breit), 2 940, 975, 948/cm.

1d) (1R, 5S, 6R, 7R)-7-Hydroxy-6-[(E)-3α-hydroxy-1-octenyl]-bicyclo[3.3.0]octan-3-on

495 mg des nach Beispiel 1c hergestellten Diols rührt man 22 Stunden mit 18 ml einer Mischung aus Essigsäure/Tetrahydrofuran/Wasser (65 + 10 + 35). Anschließend dampft man unter Zusatz von Toluol im Vakuum ein, löst den Rückstand in Methylenchlorid, schüttelt zweimal mit Sole, trocknet über Magnesiumsulfat und dampft im Vakuum ein. Den Rückstand chromatographiert man an Kieselgel mit Essigester/Pentan (9 + 1). Dabei erhält man 282 mg Titelverbindung als farbloses Öl.

IR : 3 660, 3 610, 2 940, 2 870, 1 739, 973/cm.

1e) (1R, 5S, 6R, 7R)-7-(Tetrahydropyran-2-yloxy)-6-[(E)-3α-(tetrahydropyran-2-yloxy)-1-octenyl]-bicyclo[3.3.0.]octan-3-on

Eine Lösung aus 260 mg des nach Beispiel 1d hergestellten Ketons, 0,36 ml Dihydropyran und 2,5 mg p-Toluolsulfonsäure in 11 ml Methylenchlorid rührt man 20 Minuten bei 5 °C. Anschließend verdünnt man mit Äther, schüttelt mit 4 %iger Natriumbicarbonatlösung, wäscht mit Wasser neutral, trocknet über Magnesiumsulfat und dampft im Vakuum. Man erhält 490 mg des Bistetrahydropyranyläthers, der ohne weitere Reinigung für die Wittig-Reaktion verwendet wird.

IR : 2 955, 2 862, 1 739, 970/cm.

Beispiel 2

5-{(E)-(1S, 5S, 6R, 7R)-7-Hydroxy-6-[(E)-(4RS)-3a-hydroxy-4-methyl-1-octenyl]-bicyclo[3.3.0]octan-3-yliden-pentansäure

24 ml einer 1,04 molaren Lösung von Methylsulfinylmethylnatrium in DMSO tropft man bei 15 °C unter Argon zu 6,1 g 4-Carboxylbutyltriphenylphosphoniumbromid in 12 ml DMSO abs. und rührt 30 Minuten bei Raumtemperatur. Zur roten Ylenlösung tropft man eine Lösung von 0,95 g (1R, 5S, 6R, 7R)-7-(Tetrahydropyran-2-yloxy)-6[(E)-(4RS)-4-methyl-3α-(tetrahydropyran-2-yloxy)-1-octenyl]-bicyclo-[3.3.0]octan-3-on in 6 ml abs. DMSO und rührt 2 Stunden bei 45 °C. Das Reaktionsgemisch wird auf Eiswasser gegossen, mit 10 %iger Zitronensäurelösung auf pH 4-5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Pentan (3 + 2) 0,89 g des Olefinierungsproduktes, welches man zur Abspaltung der Schutzgruppen mit 28 ml einer Mischung aus Essigsäure/Wasser/Tetrahydrofuran (65 + 35 + 10) rührt. Man dampft im Vakuum ein und chromatographiert den Rückstand an Kieselgel. Mit Methylenchlorid/Isopropanol (95 + 5) erhält man zunächst 142 mg 5-{(Z)-(1S, 5S, 6R, 7R)-7-Hydroxy-6-[(E)-(4RS)-3α-hydroxy-4-methyl-1-octenyl]-bicyclo[3.3.0]octan-3-yliden}-pentansäure sowie als polarere Komponente 230 mg der Titelverbindung als farbloses Öl.

IR : 3 610, 3 440 (breit), 2 940, 2 860, 1 712, 976/cm.

Das Ausgangsmaterial für die obige Titelverbindung wird wie folgt hergestellt :

2a) (1R, 5S, 6R, 7R)-3,3-Äthylendioxy-7-benzoyloxy-6-[(E)-(4RS)-4-methyl-3-oxo-1-octenyl]-bicyclo [3.3.0]octan

In Analogie zu Beispiel la erhält man aus 1,5 g des nach Bezugsbeispiel 1g hergestellten Aldehyds und 1,3 g 3-Methyl-2-oxoheptan-phospphonsäuredimethylester 1,62 g der Titelverbindung als farbloses Öl.

IR : 2 940, 2 860, 1 715, 1 672, 1 628, 1 275, 978, 948/cm.

2b) (1R, 5S, 6R, 7R)-3,3-Äthylendioxy-7-benzoyloxy-6-[(E)-(4RS)-3α-β-hydroxy-4-methyl-1-octenyl]-bicyclo[3.3.0]octan

Zu einer Lösung von 1,50 g des nach Beispiel 2a hergestellten Ketons in 48 ml Methanol fügt man bei −40 °C portionsweise 850 mg Natriumborhydrid und rührt 1 Stunde bei −40 °C unter Argon. Anschließend verdünnt man mit Äther, wäscht mit Wasser neutral, trocknet über Magnesiumsulfat und dampft im Vakuum ein. Durch Säulenchromatographie an Kieselgel mit Äther/Pentan (7 + 3) erhält man zunächst 520 mg der Titelverbindung (3α-Hydroxy) sowie als polarere Komponente 320 mg der isomeren 3β-Hydroxy-konfigurierten Verbindung.

IR : 3 600, 3 420 (breit), 2 940, 1 715, 1 603, 1 588, 1 278, 972, 948/cm.

2c) (1R, 5S, 6R, 7R)-3,3-Äthylendioxy-7-hydroxy-6-[(E)-(4RS)-3α-β-hydroxy-4-methyl-1-octenyl]-bicyclo[3.3.0]octan

Eine Mischung aus 510 mg des nach Beispiel 2b hergestellten α-Alkohols und 330 mg kaliumcarbonat in 35 mg Methanol rührt man 18 Stunden bei Raumtemperatur unter Argon. Anschließend engt man im Vakuum ein, verdünnt mit Äther und wäscht mit Sole neutral. Man trocknet über Magnesiumsulfat und dampft im Vakuum ein. Dabei erhält man 485 mg der Titelverbindung als farbloses Öl (Rohprodukt).

IR : 3 600, 3 430 (breit), 2 945, 976, 948/cm.

2d) (IR, 5S, 6R, 7R)-Hydroxy-6-[(E)-(4RS)-3α-β-hydroxy-4-methyl-1-octenyl]-bicyclo[3.3.0]octan-3-on

In Analogie zu Beispiel 1d erhält man aus 485 mg des nach Beispiel 2 c hergestellten Diols 295 mg der Titelverbindung als Öl.

IR : 3 600, 3 400 (breit), 2 940, 2 865, 1 740, 973/cm.

2e) (1R, 5S, 6R, 7R)-7-(Tetrahydropyran-2-yloxy)-6-[(E)-(4RS)-4-methyl-3α-(tetrahydropyran-2-yloxy)-1-octenyl]-bicyclo[3.3.0]octan-3-on

In Analogie zu Beispiel 1e erhält man aus 280 mg des nach Beispiel 2d hergestellten Ketons 460 mg des Bistetrahydropyranyläthers, der ohne weitere Reinigung für die Wittig-Reaktion verwendet wird.

IR : 2 960, 2 860, 1 740, 972/cm.

Beispiel 3

5-{(E)-(1S, 5S, 6R, 7R)-7-Hydroxy-6-[(E)-(4RS)-4-fluor-3α-hydroxy-1-octenyl]-bicyclo[3.3.0]octan-3-yliden}-pentansäure

Zu einer Lösung von 4,55 g 4-Carboxybutyl-triphenylphosphoniumbromid in 10 ml abs. DMSO tropft man bei 15 °C unter Argon 18 ml einer 1,04 molaren Lösung von Methylsulfinylmethylnatrium in DMSO und rührt 30 Minuten bei Raumtemperatur. Zur roten Ylenlösung tropft man eine Lösung von 745 mg (1R, 5S, 6R, 7R)-7-Tetrahydropyran-2-yl-oxy)-6-[(E)-(4RS)-4-fluor-3α-(tetrahydropyran-2-yloxy)-1-octenyl]-bicyclo[3.3.0]octan-3-on in 5 ml abs. DMSO und ruhrt 2 Stunden bei 45 °C. Das Reaktionsgemisch wird auf Eiswasser gegossen, mit 10 %iger Zitronensäurelösung auf pH 4-5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstands an Kieselgel erhält man mit Äther/Pentan (3 + 2) 620 mg des Olefinierungsprodukts, welches man zur Abspaltung der Schutzgruppen mit 22 ml einer Mischung aus Essigsäure/Wasser/Tetrahydrofuran (65/35/10) 20 Stunden bei 25 °C rührt. Man dampft im Vakuum ein und chromatographiert den Rückstand an Kieselgel. Mit Methylenchlorid/ Isopropanol (95 + 5) erhält man zunächst 122 mg 5-{(Z)-(1S, 5S, 6R, 7R)-7-Hydroxy-6-[(E)-(4RS)-4-fluor-3α-hydroxy-1-octenyl]-bicyclo[3.3.0]octan-3-yliden}-pentansäure sowie als polarere Komponente 208 mg der Titelverbindung als farbloses Öl.

IR : 3 600, 3 440 (breit), 2 945, 2 860, 1 713, 976/cm.

Das Ausgangsmaterial für die obige Titelverbindung wir wie folgt hergestellt :

3a) (1R, 5S, 6R, 7R)-3,3-Äthylendioxy-7-benzoyloxy-6-[(E)-(4RS)-4-fluor-3-oxo-1-octenyl]-bicyclo [3.3.0]octan

In Analogie zu Beispiel la erhält man aus 765 mg des nach Bezugsbeispiel 1g hergestellten Aldehyds und 665 mg 3-Fluor-2-oxyheptanphosphonsäuredimethylester 620 mg der Titelverbindung als farbloses Öl.

IR : 2 945, 2 860, 1 715, 1 670, 1 630, 1 276, 979, 948/cm.

3b) (1R, 5S, 6R, 7R)-3,3-Äthylendioxy-7-benzoyloxy-6-[(E)-(4RS)-4-fluor-3α-hydroxy-1-octenyl]-bicyclo[3.3.0]octan

In Analogie zu Beispiel 1b erhält man aus 410 mg des nach Beispiel 3a hergestellten Ketons und 230 mg Natriumborhydrid 146 mg der Titelverbindung als farbloses Öl.

IR : 3 600, 3 410 (breit), 2 945, 2 865, 1 715, 1 604, 1 590, 1 278, 974, 948/cm.

3c) (1R, 5S, 6R, 7R)-3,3-Äthylendioxy-7-hydroxy-6-[(E)-(4RS)-fluor-3α-hydroxy-1-octenyl]-bicyclo [3.3.0]octan

In Analogie zu Beispiel 1c erhält man aus 525 mg des nach Beispiel 3b hergestellten α-Alkohols und 340 mg Kaliumcarbonat 490 mg der Titelverbindung als Öl.

IR : 3 600, 3 400 (breit), 2 950, 2 865, 976, 948/cm.

3d) (1R, 5S, 6R, 7R)-7-Hydroxy-6-[(E)-(4RS)-4-fluor-3α-hydroxy-1-octenyl]-bicyclo[3.3.0]octan-3-on

In Analogie zu Beispiel 1d erhält man aus 470 mg des nach Beispiel 3c hergestellten Diols 285 mg der Titelverbindung als Öl.

IR : 3 600, 3 420 (breit), 2 945, 2 865, 1 740, 975/cm.

3e) (1R, 5S, 6R, 7R)-7-(Tetrahydropyran-2-yloxy)-6-[(E)-(4RS)-fluor-3α-(tetrahydropyran-2-yloxy)-1-octenyl]-bicyclo[3.3.0]octan-3-on

In Analogie zu Beispiel 1e erhält man aus 285 mg des nach Beispiel 3d hergestellten Ketons 470 mg des Bistetrahydropyranyläthers (Rohprodukt), der ohne weitere Reinigung für die Wittig-Reaktion verwendet wird.

IR : 2 960, 2 860, 1 740, 975/cm.

Beispiel 4

5-{(E)-(1S, 5S, 6R, 7R)-7-Hydroxy-6-[(E)-3α-hydroxy-4,4-methylen-1-octenyl]-bicyclo[3.3.0]octan-3-yliden}-pentansäure

36 ml einer 1,04 molaren Lösung von Methylsulfinylmethylnatrium in DMSO tropft man bei 15 °C unter Argon zu 9,2 g 4-Carboxybutyltriphenylphosphoniumbromid in 20 ml abs. DMSO und rührt 30 Min.

bei Raumtemperatur. Zur roten Ylenlösung tropft man eine Lösung von 1,45 g (1R, 5S, 6R, 7R)-7-(Tetrahydropyran-2-yloxy)-6-[(E)-4,4-methylen-3α-(tetrahydropyran-2-yloxy)-1-octenyl]-bicyclo[3.3.0]octan-3-on in 10 ml DMSO und rührt 2 Std. bei 45 °C. Das Reaktionsgemisch wird auf Eiswasser gegossen, mit 10 %iger Zitronensäurelösung auf pH 5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Pentan (3 + 2) 1,38 g des Olefinierungsprodukts, welches man zur Abspaltung der Schutzgruppen mit 35 ml einer Mischung aus Essigsäure/Wasser/Tetrahydrofuran (65 + 35 + 10) rührt. Man dampft im Vakuum ein und chromatographiert den Rückstand an Kieselgel. Mit Methylenchlorid/Isopropanol (95 + 5) erhält man zunächst 210 mg 5-{(Z)-(1S, 5S, 6R, 7R)-7-Hydroxy-6-[(E)-3α-hyroxy-4,4,methylen-1-octenyl[-bicyclo[3.3.0]octan-3-yliden}-pentansäure sowie als polarere Komponente 295 mg der Titelverbindung als farbloses Öl.

IR : 3 600, 3 450 (breit), 2 945, 2 865, 1 712, 976/cm.

Das Ausgangsmaterial für die obige Titelverbindung wird wie folgt hergestellt :

4a) (1R, 5S, 6R, 7R)-3,3-Athylendioxy-7-benzoyloxy-6-[(E)-4,4-methylen-3-oxo-1-octenyl]-bicyclo-[3.3.0]octan

In Analogie zu Beispiel la erhält man aus 1,48 g des nach Bezugs-beispiel 1 g hergestellten Aldehyds und 1,3 g 3,3-methylen-2-oxo-heptanphosphonsäuredimethylester 1,55 g der Titelverbindung als farbloses Öl.

IR : 2 940, 2 860, 1 715, 1 670, 1 630, 1 275/978/cm.

4b) (1R, 5S, 6R, 7R)-3,3-Äthylendioxy-7-benzoyloxy-6-[(E)-3α-hydroxy-4,4-methylen-1-octenyl]-bicyclo [3.3.0]octan

In Analogie zu Beispiel Ib erhält man aus 1,45 g des nach Beispiel 4a hergestellten Ketons und 850 mg Natriumborhydrid 510 mg der Titelverbindung als farbloses Öl.

IR : 3 600, 3 400 (breit), 2 945, 2 860, 1 715, 1 603, 1 590, 1 277, 973, 948/cm.

4c) (1R, 5S, 6R, 7R)-3,3-Äthylendioxy-7-hydroxy-6-[(E)-3α-hydroxy-4,4-methylen-1-octenyl]-bicyclo [3.3.0]octan

In Analogie zu Beispiel Ic erhält man aus 490 mg des nach Beispiel 4b hergestellten α-Alkohols und 320 mg Kaliumcarbonat 470 mg der Titelverbindung als farbloses Öl.

IR : 3 600, 3 400 (breit), 2 940, 2 860, 976/cm.

4d) (1R, 5S, 6R, 7R)-7-Hydroxy-6-[(E)-3α-hydroxy-4,4-methylen-1-octenyl]-bicyclo[3.3.0]octan-3-on

In Analogie zu Beispiel Id erhält man aus 470 mg des nach Beispiel 4c hergestellten Diols 280 mg der Titelverbindung als Öl.

IR : 3 600, 3 400 (breit), 2 945, 2 860, 1 740, 974/cm.

4e) (1R, 5S, 6R, 7R)-7-(Tetrahydropyran-2-yloxy)-6-[(E)-4,4-methylen-aα-(tetrahydropyran-2-yloxy)-1-octenyl]-bicyclo[3.3.0]octan-3-on

In Analogie zu Beispiel Ie erhält man aus 270 mg des nach Beispiel 4d hergestellten Ketons 440 mg des Bistetrahydropyranyläthers, der ohne weitere Reinigung für die Wittig-Olefinierung verwendet wird.

IR : 2 960, 2 860, 1 739, 975/cm.

## Beispiel 5

5-{(E)-(1S, 5S, 6R, 7R)-7-Hydroxy-6-[(E)-3α-hydroxy-1-nonenyl]-bicyclo[3.3.0]octan-3-yliden}-pentansäure

Zu einer Lösung von 3,34 g 4-Carboxybutyl-triphenylphosphoniumbromid in 6,5 ml abs. DMSO tropft man bei 15 °C unter Argon 13,2 ml einer 1,04 M Lösung von Methylsulfinylmethylnatrium in DMSO. Nach 15 Minuten tropft man zu dieser Ylenlösung eine Lösung von 500 mg (1R, 5S, 6R, 7R)-7-(Tetrahydropyran-2-yloxy)-6-[(E)-3α-(tetrahydropyran-2-yloxy)-1-nonenyl]-bicyclo[3.3.0]octan-3-on in 3 ml abs. DMSO und erwärmt 2 Stunden auf 45-50 °C. Man gibt dann auf Eiswasser, säuert mit Zitronensäure auf pH 5 an und extrahiert mit Methylenchlorid. Der Extrakt wird mit Sole geschüttelt, über Magnesiumsulfat getrocknet und in Vakuum eingedampft. Chromatographie des Rückstandes an Kieselgel mit Äther/Pentan-Gemisches liefert 435 mg eines gelben Öles, das zur Abspaltung der Schutzgruppen mit 15 ml einer Mischung aus Essigsäure/Wasser/Tetrahydrofuran (65/35/10) 16 Stunden bei 40 °C rührt. Nach Eindampfen der Lösung wird der Rückstand an Kieselgel mit Methylenchlorid/Isopropanol (95 + 5)

chromatographiert. Man erhält 80 mg 5-{(Z)-(1S, 5S, 6R, 7R)-7-Hydroxy-6-[(E)-3α-hydroxy-1-nonenyl]-bicyclo[3.3.0]octan-3-yliden}-pentansäure und als polarere Komponente 120 mg der Titelverbindung als farbloses zähes Öl.

IR : 3 600, 3 455 (breit), 2 945, 2 865, 1 710, 978/cm.

Das Ausgangsmaterial für die Titelverbindung wird wie folgt hergestellt :

5a) (1R, 5S, 6R, 7R)-3,3-Äthylendioxy-7-benzoyloxy-6-[(E)-3-oxo-1-nonenyl]-bicyclo[3.3.0]octan

Man suspensiert 252 mg Natriumhydrid (55 %ig) in 25 ml abs. Dimethoxyäthan und tropft bei 15 °C 1,39 g 2-Oxo-octylphosphonsäuredimethylester in 10 ml Dimethoxyäthan gelöst zu. Man rührt 10 Minuten, versetzt mit 245 mg Lithiumchlorid und tropft nach einer Stunde bei −20 °C eine Lösung von 1,51 g des nach Bezugsbeispiel Ig hergestellten Aldehyds in 25 ml abs. Dimethoxyäthan zu. Anschließend rührt man 2 Stunden bei 10-15 °C, gibt auf 250 ml gesättigte Ammoniumchloridlösung, extrahiert mehrmals mit Äther, wäscht den organischen Extrakt mit Sole, trocknet über Magnesiumsulfat und dampft im Vakuum ein. Den Rückstand reinigt man durch Chromatographie an Kieselgel mit Äther-Hexan-Gemischen. Man erhält 1,49 g des o.a. Ketons als Öl.

IR : 2 945, 2 860, 1 715, 1 670, 1 630, 1 275, 978, 948/cm.

5b) (1R, 5S, 6R, 7R)-3,3-Äthylendioxy-7-benzoyloxy-6-[(E)-3α-hydroxy-1-nonenyl]-bicyclo[3.3.0] octan

Zu einer Lösung von 1,44 g des nach Beispiel 5a hergestellten Ketons in 40 ml Methanol gibt man unter Rühren bei −40 °C portionsweise 800 mg Natriumborhydrid, rührt 1 Stunde bei −40 °C, verdünnt mit 200 ml Äther, wäscht mit Sole neutral, trocknet über Magnesiumsulfat und dampft im Vakuum ein. Das Gemisch der epimeren Alkohole trennt man durch Chromatographie an Kieselgel mit Hexan/Äther-Gemischen. Man erhält als impolare Komponente 576 mg des gewünschten (3S)-Alkohols als Öl, sowie als polarere Komponente 490 ml des (3R)-Alkohols, ebenfalls als Öl.

IR : 3 600, 3 400 (breit), 2 945, 1 715, 1 602, 1 588, 1 275, 975, 948/cm.

5c) (1R, 5S, 6R, 7R)-3,3-Äthylendioxy-7-hydroxy-6-[(E)-3α-hydroxy-1-nonenyl]-bicyclo[3.3.0]octan

500 mg des (3S)-Alkohols aus Beispiel 5b werden in 35 ml Methanol mit 315 mg Kaliumcarbonat 16 Std. bei 20 °C gerührt. Nach Einengen in Vakuum wird mit 200 ml Äther verdünnt, mit Wasser neutral gewaschen, über Magnesiumsulfat getrocknet und im Vakuum eingedampft. Das Rohprodukt wird ohne weitere Reinigung in die nächste Stufe eingesetzt.

5d) (1R, 5S, 6R, 7R)-7-Hydroxy-6-[(E)-3α-hydroxy-1-nonenyl]-bicyclo[3.3.0]octan-3-on

Das Rohprodukt aus Beispiel 5c rührt man 22 Std. mit 20 ml einer Mischung aus Essigsäure/Wasser/Tetrahydrofuran (65/35/10), dampft unter Zusatz von Toluol im Vakuum ein und chromatographiert den Rückstand an Kieselgel mit Essigester/Xexan-Gemischen. Man erhält 270 mg des o.a. Ketons als Öl.

IR : 3 600, 2 945, 2 870, 1 740, 975/cm.

5e) (1R, 5S, 6R, 7R)-7-(Tetrahydropyran-2-yloxy)-6-[(E)-3α-(tetrahydropyran-2-yloxy)-1-nonenyl]-bicyclo[3.3.0]octan-3-on

250 mg des Ketons aus Beispiel 5d werden in 10 ml Methylenchlorid mit 0,35 ml Dihydropyran und 2,5 mg p-Toluolsulfonsäure 30 Min. bei 0-5 °C gerührt. Anschließend verdünnt man mit 100 ml Methylenchlorid, schüttelt mit Natriumbicarbonatlösung und Sole, trocknet über Magnesiumsulfat und dampft im Vakuum ein. Man erhält 475 mg des Bistetrahydropyranyläthers als gelbes Öl.

IR : 2 955, 2 860, 1 740, 972/cm.

## Beispiel 6

5-{(E)-(1S, 5S, 6R, 7R)-7-Hydroxy-6-[(E)-(4RS)-3α-hydroxy-4-methyl-1-nonenyl]-bicyclo[3.3.0]octan-3-yliden}-pentansäure

In Analogie zu Beispiel 5 erhält man aus 420 mg (1R, 5S, 6R, 7R)-7-(Tetrahydropyran-2-yloxy)-6-[(E)-(4RS)-4-methyl-3α-(tetrahydropyran-2-yloxy-1-nonenyl]-bicyclo[3.3.0]octan-3-on 95 mg der Titelverbindung und 85 mg des Z-Isomeren 5-{(Z)-(1S, 5S, 6R, 7R)-7-Hydroxy-6-[(E)-(4RS)-3α-hydroxy-4-methyl-1-nonenyl]-bicyclo[3.3.0.]octan-3-yliden}-pentansäure.

IR : (E-Isomeren) : 3 600, 3 450 (breit), 2 945, 2 860, 1 710, 973/cm.

Das Ausgangsmaterial für die Titelverbindung wird wie folgt hergestellt :

6a) (1R, 5S, 6R, 7R)-3,3-Äthylendioxy-7-benzoyloxy-6-[(E)-4-methyl-3-oxo-1-nonenyl]-bicyclo [3.3.0]octan

In Analogie zu Beispiel 5a erhält man aus 2g (1R, 5S, 6R, 7R)-3,3-Äthylendioxy-7-benzoyloxy-6-formyl-bicyclo[3.3.0]octan mit 3-Methyl-2-oxo-octylphosphonsäuredimethylester 2,01 g des o.a. Ketons als zähes Öl.
IR : 2 950, 2 860, 1 715, 1 670, 1 630, 1 602, 1 275, 978, 948/cm.

6b) (1R, 5S, 6R, 7R)-3,3-Äthylendioxy-7-benzoyloxy-6-[(E)-(4RS)-3α-hydroxy-4-methyl-1-nonenyl]-bicyclo[3.3.0]octan

In Analogie zu Beispiel 5b erhält man aus 1,95 g des nach Beispiel 6a hergestellten Ketons 800 mg des o.a. (3R)-α-Alkohols und als polareren Anteil 730 mg des (3S)-ß-Alkohols.
IR : 3 600, 3 400 (breit), 2 950, 1 715, 1 602, 1 588, 1 270, 978, 948/cm.

6c) (1R, 5S, 6R, 7R)-3,3-Äthylendioxy-7-hydroxy-6-[(E)-(4RS)-3α-3-hydroxy-4-methyl-1-nonemyl]-bicyclo[3.3.0]octan

In Analogie zu Beispiel 5c erhält man aus 790 mg des nach Beispiel 6b hergestellten (3R)-α-Alkohols 750 mg des o.a. Diols als Rohprodukt.

6d) (1R, 5S, 6R, 7R)-7-Hydroxy-6-[(E)-(4RS)-3α-hydroxy-4-methyl-1-nonenyl]-bicyclo[3.3.0]octan-3-on

In Analogie zu Beispiel 5d erhält man aus 730 mg des nach Beispiel 6c hergestellten Diols 420 mg des o.a. Ketons als farbloses Öl.
IR : 3 600, 2 950, 2 870, 1 740, 978/cm.

6e) (1R, 5S, 6R, 7R)-7-(Tetrahydropyran-2-yloxy)-6-[(E)-(4RS)-4-methyl-3α-(tetrahydropyran-2-yloxy)-1-nonenyl]-bicyclo[3.3.0]octan-3-on

In Analogie zu Beispiel 5e erhält man aus 700 mg des nach Beispiel 6d hergestellten Ketons 950 mg des o.a. Bistetrahydropyranyläthers als Öl.
IR : 2 950, 2 860, 1 740, 978/cm.

## Beispiel 7

5-{(E)-(1S, 5S, 6R, 7R)-7-Hydroxy-6-[(E)-3α-hydroxy-4-phenyl-1-butenyl]-bicyclo[3.3.0]octan-3-yliden}-pentansäure

Eine Lösung von 2,21 g 4-Carboxybutyl-triphenylphosphoniumbromid in 5 ml abs. DMSO versetzt man bei 15 °C mit 9,5 ml einer 1,04 M Lösung von Methylsulfinylmethylnatrium in DMSO. Nach 15 Min. gibt man 440 mg (1R, 5S, 6R, 7R)-7-(Tetrahydropyran-2-yloxy)-6-[(E)-4-phenyl-3α-(tetrahydropyran-2-yloxy)-1-butenyl]-1-butenyl]-bicyclo[3.3.0]octan-3-on gelöst in 3 ml abs. DMSO zu und rührt 2 Std. bei 50 °C, gibt dann auf Eiswasser, stellt mit Zitronensäure auf pH 4,5 ein und extrahiert mit Methylenchlorid. Der Extrakt wird mit Sole geschüttelt, über Magnesiumsulfat getrocknet und im Vakuum eingedampft. Nach Reinigung durch Chromatographie an Kieselgel mit Hexan/Äther-Gemischen setzt man zur Abspaltung der Schutzgruppen mit Essigsäure um (analog Beispiel 5) und erhält nach chromatographischer Reinigung an Kieselgel mit Methylenchlorid/Isopropanol (95 + 5) 75 mg 5-{(Z)-(1S, 5S, 6R, 7R)-7-Hydroxy-6-[(E)-3α-hydroxy-4-phenyl-1-butenyl]-bicyclo[3.3.0]octan-3-yliden}-pentansäure und als polarere Komponente 110 mg der Titelverbindung als farbloses Öl.
IR : 3 600, 3 450 (breit), 2 945, 2 860, 1 710, 1 602, 978/cm.
Das Ausgangsmaterial für die Titelverbindung wird wie folgt hergestellt :

7a) (1R, 5S, 6R, 7R)-3,3-Äthylendioxy-7-benzoyloxy-6-[(E)-3-oxo-4-phenyl-1-butenyl]-bicyclo [3.3.0]octan

In Analogie zu Beispiel 5a erhält man aus 2,5 g (1S, 5S, 6R, 7R)-3,3-Äthylendioxy-7-benzoyloxy-6-formyl-bicyclo[3.3.0]octan durch Umsetzung mit dem Natriumsalz von 2-Oxo-3-phenyl-propylphosphonsäuredimethylester 2,45 g des o.a. Ketons als Öl.
IR : 2 955, 2 870, 1 712, 1 670, 1 632, 1 600, 1 275, 975, 948/cm.

7b) (1R, 5S, 6R, 7R)-3,3-Äthylendioxy-7-benzoyloxy-6-[(E)-3α-hydroxy-4-phenyl-1-butenyl]-bicyclo[3.3.0]octan

In Analogie zu Beispiel 5b erhält man aus 2,40 g des nach Beispiel 7a hergestellten Ketons 1,05 g des

o.a. (3S)-α-Alkohols und als polareren Anteil 0,95 g des (3R)-β-Alkohols.
IR : 3 600, 3 400 (breit), 2 950, 2 865, 1 712, 1 602, 1 588, 1 270, 978, 948/cm.

7c) (1R, 5S, 6R, 7R)-3,3-Äthylendioxy-7-hydroxy-6-[(E)-3d-hydroxy-4-phenyl-1-butenyl]-bicyclo [3.3.0]octan

In Analogie zu Beispiel 5c erhält man aus 1,02 g des nach Beispiel 7b hergestellten (3S)-α-Alkohols 800 mg des o.a. Diols als Rohprodukt.

7d) (1R, 5S, 6R, 7R)-7-Hydroxy-6-[(E)-3α-hydroxy-4-phenyl-1-butenyl]-bicyclo[3.3.0]octan-3-on

In Analogie zu Beispiel 5d erhält man aus 800 mg des nach Beispiel 7c hergestellten Diols 530 mg des o.a. Ketons als farbloses Öl.
IR : 3 600, 2 950, 2 865, 1 738, 1 602, 975/cm.

7e) (1R, 5S, 6R, 7R)-7-(Tetrahydropyran-2-yloxy)-6-[(E)-4-phenyl-3α-(tetrahydropyran-2-yloxy)-1-butenyl]-bicyclo[3.3.0]octan-3-on

In Analogie zu Beispiel 5e erhält man aus 500 mg des nach Beispiel 7d hergestellten Ketons 700 mg des o.a. Bistetrahydropyranyläthers als Öl.
IR : 2 950, 2 860, 1 738, 1 602, 976/cm.

Beispiel 8

5-{(E)-(1S, 5S, 6R, 7R)-7-Hydroxy-6-[(4RS)-3α-hydroxy-4-methyl-1-octyl]-bicyclo[3.3.0]octan-3-yliden}-pentansäure

Eine Ylenlösung, hergestellt aus 3g 4-Carboxybutyl-triphenylphosphoniumbromid analog Beispiel 5, versetzt man mit 450 mg (1R, 5S, 6R, 7R)-7-(Tetrahydropyran-2-yloxy)-6-[(4RS)-4-methyl-3α-(tetrahydropyran-2-yloxy)-1-octyl]-bicyclo[3.3.0]octan-3-on gelöst in 3 ml abs. DMSO und rührt 2 Stunden bei 50 °C. Man verdünnt mit Eiswasser, säuert mit Zitronensäure auf pH 4,5 an und extrahiert mehrmals mit Methylenchlorid. Die Extrakte werden vereinigt, mit Sole geschüttelt, über Magnesiumsulfat getrocknet und im Vakuum eingedampft. Zur Entfernung der Schutzgruppen rührt man das Rohprodukt 6 Stunden bei 45 °C mit 20 ml einer Mischung aus Essigsäure/Wasser/Tetrahydrofuran (65/35/10). Nach Eindampfen zur Trockne wird der Rückstand an Kieselgel mit Methylenchlorid/1-5 % Isopropanol chromatographiert. Man erhält 80 mg 5-{(Z)-(1S, 5S, 6R, 7R)-7-Hydroxy-6-[(4RS)-3α-hydroxy-4-methyl-1-octyl]-bicyclo[3.3.0]octan-3-yliden}-pentansäure und als polarere Komponente 110 mg der Titelverbindung als farbloses Öl.
IR : 3 600, 3 450 (breit), 2 950, 2 860, 1 710/cm.
Das Ausgangsmaterial für die Titelverbindung wird wie folgt hergestellt :

8a) (1R, 5S, 6R, 7R)-7-(Tetrahydropyran-2-yloxy)-6-[(4RS)-4-methyl-3α-(tetrahydropyran-2-yloxy)-1-octyl]-bicyclo[3.3.0]octan-3-on

Eine Lösung von 1g (1R, 5S, 6R, 7R)-7-(Tetrahydropyran-2-yloxy)-6-[(E)-(4RS)-4-methyl-3α-(tetrahydropyran-2-yloxyl)-1-octenyl]-bicyclo[3.3.0]octan-3-on in 25 ml Essigester schüttelt man mit 100 mg Palladium auf Kohle (10 %ig) unter einer Wasserstoffatmosphäre ca. 1 Std. bis zur Aufnahme von 1 Mol Wasserstoff pro Mol Substrat. Nach Filtrieren und Abdampfen des Lösungsmittels erhält man die o.a. Verbindung als hellgelbes Öl.
IR : 2 960, 2 865, 1 740/cm.

Beispiel 9

5-{(E)-(1S, 5S, 6R, 7R)-7-Hydroxy-6-[3α-hydroxy-1-nonyl]-bicyclo[3.3.0]octan-3-yliden}-pentansäure

Eine Ylenlösung, hergestellt aus 3,5 g 4-Carboxybutyl-triphenylphosphoniumbromid analog Beispiel 5, versetzt man mit 500 mg (1R, 5S, 6R, 7R)-7-(Tetrahydropyran-2-yloxy)-6-[3α-(tetrahydropyran-2-yloxy)-1-nonyl]-bicyclo[3.3.0]octan-3-on gelöst in 3 ml abs. DMSO und rührt 2 Std. bei 50 °C. Nach Verdünnen mit Eiswasser und ansäuern auf pH 4,5 mit Zitronensäure extrahiert man mehrfach mit Methylenchlorid. Die vereinigten Extrakte werden mit Sole geschüttelt, über Magnesiumsulfat getrocknet und im Vakuum eingedampft. Zur Entfernung der Schutzgruppen rührt man das Rohprodukt 6 Std. bei 45 °C mit 20 ml einer Mischung aus Essigsäure/Wasser/Tetrahydrofuran (65/35/10). Nach Eindampfen zur Trockne wird der Rückstand an Kieselgel mit Methylenchlorid/1-5 % Isopropanol chromatographiert. Man erhält 100 mg 5-(Z)-(1S, 5S, 6R, 7R)-7-Hydroxy-6-[3α-hydroxy-1-nonyl]-bicyclo[3.3.0]octan-3-yliden-pentansäure und als polarere Komponente 120 mg der Titelverbindung als farbloses Öl.

IR : 3 600, 3 455 (breit), 2 950, 2 865, 1 710/cm.
Das Ausgangsmaterial für die Titelverbindung wird wie folgt hergestellt :

9a) (1R, 5S, 6R, 7R)-7-(Tetrahydropyran-2-yloxy)-6-[3α-(tetrahydropyran-2-yloxy)-1-nonyl]-bicyclo [3.3.0]octan-3-on

800 mg (1R, 5S, 6R, 7R)-7-(Tetrahydropyran-2-yloxy)-6-[(E)-(3S)-3-(tetrahydropyran-2-yloxy)-1-nonenyl]-bicyclo[3.3.0]octan-3-on (Herstellung siehe Beispiel 5e) gelöst in 20 ml Essigester werden mit 80 mg Palladium auf Kohle (10 %ig) unter einer Wasserstoffatmosphäre geschüttelt bis zu einer Aufnahme von 1 Mol Wasserstoff pro Mol Substrat. Nach Filtrieren und Abdampfen des Lösungsmittels erhält man die o.a. Verbindung als Öl
IR : 2 965, 2 865, 1 740/cm.

## Beispiel 10

5-{(E)-(1S, 5S, 6R, 7R)-7-Hydroxy-6-[(E)-3α-hydroxy-oct-1-en-6-inyl]-bicyclo[3.3.0]octan-3-yliden}-pentansäure

Zu einer Lösung von 2,66 g 4-Carboxybutyltriphenylphosphoniumbromid in 6 ml abs. DMSO tropft man bei 15 °C unter Argon 10,6 ml einer 1,04 molaren Lösung von Methylsulfinylmethylnatrium in DMSO und rührt 30 Min. bei Raumtemperatur. Zur roten Ylenlösung tropft man eine Lösung von 430 mg (1R, 5S, 6R, 7R)-7-(Tetrahydropyran-2-yloxy)-6-[(E)-3α-(tetrahydropyran-2-yloxy)-oct-1-en-6-inyl]-bicyclo [3.3.0]octan-3-on in 3 ml abs. DMSO und rührt 2 Std. bei 45 °C. Das Reaktionsgemisch wird auf Eiswasser gegossen, mit 10 %iger Zitronensäurelösung auf pH 4-5 angesäuert und 3x mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Pentan (3 + 2) 445 mg des Olefinierungsprodukts, welches man zur Abspaltung der Schutzgruppen mit 15 ml einer Mischung aus Essigsäure/Wasser/Tetrahydrofuran (65 + 35 + 10) 20 Std. bei 25 °C rührt. Man dampft im Vakuum ein und chromatographiert den Rückstand an Kieselgel. Mit Methylenchlorid/Isopropanol (95 + 5) erhält man zunächst 72 mg 5-{(E)-(1S, 5S, 6R, 7R)-7-Hydroxy-6[(Z)-3α-hydroxy-oct-1-en-6-inyl]-bicyclo[3.3.0]octan-3-yliden}-pentansäure sowie als polarere Komponente 121 mg der Titelverbindung als farbloses Öl.
IR : 3 600, 3 420 (breit), 2 945, 2 860, 1 712, 976/cm.
Das Ausgangsmaterial für die oblige Titelverbindung wird wie folgt hergestellt :

10a) (1R, 5S, 6R, 7R)-3,3-Äthylendioxy-7-benzoyloxy-6-[(E)-3-oxo-oct-1-en-6-inyl]-bicyclo [3.3.0]octan

In Analogie zu Beispiel la erhält man aus 560 mg des nach Bazugsbeispiel 1 g hergestellten Aldehyds und 0,5 g 2-Oxo-hept-5-in-phosphonsäuredimethylester 0,62 g der Titelverbindung als farbloses Öl.
IR : 2 945, 2 860, 1 714, 1 672, 1 630, 1 275, 978, 948/cm.

10b) (1R, 5S, 6R, 7R)-3,3-Äthylendioxy-7-benzoyloxy-6-[(E)-3α-hydroxy-oct-1-en-6-inyl]-bicyclo [3.3.0]octan

In Analogie zu Beispiel 1 b erhält man aus 400 mg des nach Beispiel 10a hergestellten Ketons und 220 mg Natriumborhydrid 135 mg der Titelverbindung als farbloses Öl.
IR : 3 600, 3 410 (breit), 2 940, 2 860, 1 715, 1 603, 1 590, 1 278, 972, 948/cm.

10c) (1R, 5S, 6R, 7R)-3,3-Äthylendioxy-7-hydroxy-6-[(E)-3α-hydroxy-oct-1-en-6-inyl]-bicyclo [3.3.0]octan

In Analogie zu Beispiel 1c erhält man aus 240 mg des nach Beispiel 10b hergestellten α-Alkohols und 165 mg Kaliumcarbonat 230 mg der Titelverbindung als farbloses Öl (Rohprodukt).
IR : 3 600, 3 440 (breit), 2 945, 2 860, 974, 948/cm.

10d) (1R, 5S, 6R, 7R)-7-Hydroxy-6-[(E)-3α-hydroxy-oct-1-en-6-inyl]-bicyclo[3.3.0]octan-3-on

In Analogie zu Beispiel Id erhält man aus 230 mg des nach Beispiel 10c hergestellten Diols 141 mg der Titelverbindung als farbloses Öl.
IR : 3 640, 3 610, 2 945, 2 865, 1 740, 974/cm.

10e) (1R, 5S, 6R, 7R)-7-(Tetrahydropyran-2-yloxy)-6-[(E)-3α-(tetrahydropyran-2-yloxy)-oct-1-en-6-inyl]-bicyclo[3.3.0]octan-3-on

In Analogie zu Beispiel le erhält man aus 130 mg des nach Beispiel 10d hergestellten Ketons und 0,18 ml Dihydropyran 230 mg des Bistetrahydropyranyläthers, der ohne weitere Reinigung für die Wittig-Reaktion verwendet wird.

IR : 2 960, 2 860, 1 740, 972/cm.

Beispiel 11

5-{(E)-(1S, 5S, 6R, 7R)-7-Hydroxy-6-[(E)-(4RS)-3α-hydroxy-4-methyl-oct-1-en-6-inyl]-bicyclo [3.3.0]octan-3-yliden}-pentansäure

Zu einer Lösung von 5,3 g 4-Carboxybutyl-triphenylphosphoniumbromid in 12 ml abs. DMSO tropft man bei 15 °C unter Argon 21,3 ml einer 1,04 molaren Lösung von Methylsulfinylmethylnatrium in DMSO und rührt 30 Min. bei Raumtemperatur. Zur roten Ylenlösung tropft man eine Lösung von 870 mg (1R, 5S, 6R, 7R)-7-(Tetrahydropyran-2-yloxy)-6-[(E)-(4RS)-4-methyl-3α-(tetrahydropyran-2-yloxy)-oct-1-en-6-inyl]-bicyclo[3.3.0]octan-3-on in 6 ml abs. DMSO und rührt 2 Std. bei 45 °C. Das Reaktionsgemisch wird auf Eiswasser gegossen, mit 10% iger Zitronensäurelösung auf pH 5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Pentan (3 + 2) 940 mg des Olefinierungsprodukts, welches man zur Abspaltung der Schutzgruppen mit 30 ml einer Msichung aus Essigsäure/Wasser/Tetrahydrofuran (65 + 35 + 10) 20 Std. bei 25 °C rührt. Man dampft im Vakuum ein und chromatographiert den Rückstand an Kieselgel. Mit Methylenchlorid/Isopropanol (95 + 5) 165 mg 5-{(Z)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure sowie als polarere Komponente 253 mg der Titelverbindung als farbloses Öl.

IR : 3 600, 3 400 (breit), 2 940, 2 860, 1 712, 975/cm.

Das Ausgangsmaterial für die oblige Titelverbindung wird wie folgt hergestellt :

11a) (1R, 5S, 6R, 7R)-3,3-Äthylendioxy-7-benzoyloxy-6-[(E)-(4RS)-4-methyl-3-oxo-oct-1-en-6-inyl]-bicyclo[3.3.0]octan

In Analogie zu Beispiel la erhält man aus 1,3 g des nach Bezugsbeispiel 1 g hergestellten Aldehyds und 1g 3-Methyl-2-oxo-hept-5-in-phosphonsäure-dimethylester 1,45 g der Titelverbindung als Öl.

IR : 2 940, 2 860, 1 714, 1 670, 1 629, 1 275, 978, 948/cm.

11b) (1R, 5S, 6R, 7R)-3,3-Äthylendioxy-7-benzoyloxy-6-[(E)-(4RS)-3α-hydroxy-4-methyl-oct-1-en-6-inyl]-bicyclo[3.3.0]octan

In Analogie zu Beispiel 1b erhält man aus 810 mg des nach Beispiel lla hergestellten Ketons und 450 mg Natriumborhydrid 380 mg der Titelverbindung als farbloses Öl.

IR : 3 600, 3 400 (breit), 2 945, 2 860, 1 715, 1 602, 1 589, 1 278, 973, 948/cm.

11c) (1R, 5S, 6R, 7R)-3,3-Äthylendioxy-7-hydroxy-6-[(E)-(4RS)-3α-hydroxy-4-methyl-oct-1-en-6-inyl]-bicyclo[3.3.0]octan-3-on

In Analogie zu Beispiel 1c erhält man aus 500 mg des nach Beispiel 11b hergestellten α-Alkohols und 340 mg Kaliumcarbonat 465 mg der Titelverbindung als Öl (Rohprodukt).

IR : 3 600, 3 400 (breit), 2 940, 2 860, 976, 948/cm.

11d) (1R, 5S, 6R, 7R)-7-Hydroxy-6-[(E)-(4RS)-3α-hydroxy-4-methyl-oct-1-en-6-inyl]-bicyclo [3.3.0]octan-3-on

In Analogie zu Beispiel 1d erhält man aus 455 mg des nach Beispiel 11c hergestellten Diols 295 mg der Titelverbindung als farbloses Öl.

IR : 3 600, 2 945, 2 860, 1 740, 974/cm.

11e) (1R, 5S, 6R, 7R)-7-(Tetrahydropyran-2-yloxy)-6-[(E)-(4RS)-4-methyl-3α-(tetrahydropyran-2-yloxy)-oct-1-en-6-inyl]-bicyclo[3.3.0]octan-3-on

In Analogie zu Beispiel le erhält man aus 270 mg des nach Beispiel 11d hergestellten Ketons und 0,38 ml Dihydropyran 460 mg des Bistetrahydropyranyläthers, der ohne weitere Reinigung für die Wittig-Reaktion verwendet wird.

IR : 2 960, 2 865, 1 738, 975/cm.

Beispiel 12

5-{(E)-(1S, 5S, 6R, 7R)-7-Hydroxy-6-[(E)-3αβ-hydroxy-3-methyl-oct-1-en-6-inyl]-bicyclo[3.3.0]octan-3-yliden}-pentansäure

Zu einer Lösung von 1,50 g 4-Carboxybutyl-triphenylphosphoniumbromid in 5 ml abs. DMSO tropft man bei 15 °C unter Argon 6 ml einer 1,04 M Lösung von Methylsulfinylmethylnatrium in DMSO. Nach 15 Minuten versetzt man mit 250 mg (1S, 5S, 6R, 7R)-7-(Tetrahydropyran-2-yloxy)-6-[(E)-3αβ-3-methyl-3-(tetrahydropyran-2-yloxy)-oct-1-en-6-inyl]-bicyclo[3.3.0]octan-3-on gelöst in 3 ml abs. DMSO und rührt 2 Stunden bei 50 °C. Nach Verdünnen mit Eiswasser und ansäuern auf pH 4,5 mit verdünnter Zitronensäurelösung extrahiert man mehrfach mit Methylenchlorid, wäscht den Extrakt mit Sole, trocknet über Magnesiumsulfat und dampft im Vakuum ein. Nach Chromatographie des Rückstandes an Kieselgel erhält man mit Äther/Pentan (1:1) 270 mg eines Rohproduktes, das man zur Abspaltung der Schutzgruppen mit 8 ml einer Mischung aus Essigsäure/Wasser/Tetrahydrofuran (65/35/10) 20 Stunden bei 25 °C rührt. Man dampft im Vakuum ein und chromatographiert den Rückstand an Kieselgel mit Methylenchlorid/1-5 % Isopropanol und erhält zunächst 35 mg 5-{(Z)-(1S, 5S, 6R, 7R)-7-Hydroxy-6-[(E)-3αβ-hydroxy-3-methyl-oct-1-en-6-inyl]-bicyclo[3.3.0]octan-3-yliden}-pentansäure und als polarere Komponente 55 mg der Titelverbindung als farbloses Öl.

IR : 3 600, 3 420 (breit), 2 950, 2 865, 1 710, 978/cm.

12a) (1R, 5S, 6R, 7R)-3,3-Äthylendioxy-7-benzoyloxy-6-[(E)-3αβ-hydroxy-3-methyl-oct-1-en-6-inyl]-bicyclo[3.3.0]octan

Zu einer Lösung von 4 g (1R, 5S, 6R, 7R)-3,3-Äthylendioxy-7-benzoyloxy-6-[(E)-3-oxo-oct-1-en-6-inyl]-bicyclo[3.3.0]octan (Herstellung siehe Beispiel 10a) in 150 ml abs. Tetrahydrofuran tropft man bei − 60 °C 30 ml einer ätherischen Lösung von Methylmagnesiumbromidlösung (hergestellt aus 0,1 Mol Magnesium), rührt 15 Min. und gießt anschließend in 200 ml gesättigte Ammoniumchloridlösung ein, rührt 10 Min. bei 20 °C, extrahiert 4x mit je 75 ml Äther, wäscht die vereinigten Extrakte 2x mit je 30 ml Sole, trocknet über Magnesiumsulfat und dampft im Vakuum ein. Nach Reinigung durch Säulenchromatographie an Kieselgel mit Hexan/Essigester-Gemischen erhält man 3,5 g des o.a. Alkohols als Öl.

IR : 3 600, 3 450 (breit), 2 960, 2 865, 1 715, 1 602, 1 588, 1 275, 976, 948/cm.

12b) (1R, 5S, 6R, 7R)-3,3-Äthylendioxy-7-hydroxy-6-[(E)-3αβ-hydroxy-3-methyl-oct-1-en-6-inyl]-bicyclo[3.3.0]

Eine Lösung von 3,3 g des nach Beispiel 12a hergestellten Alkohols in 300 ml Methanol rührt man 16 Std. bei 25 °C mit 2,5 g Kaliumcarbonat. Anschließend verdampft man das Methanol im Vakuum, verteilt den Rückstand zwischen Methylenchlorid und Wasser, trocknet die organische Phase über Magnesiumsulfat und dampft im Vakuum ein. Den Rückstand filtriert man über Kieselgel mit Hexan/Essigester-Gemischen und erhält 2,20 g des o.a. Diols als farbloses Öl.

IR : 3 600, 3 450 (breit), 2 965, 2 870, 978, 948/cm.

12c) (1R, 5S, 6R, 7R)-7-Hydroxy-6-[(E)-3αβ-hydroxy-3-methyl-oct-1-en-6-inyl]-bicyclo[3.3.0]-3-on

2 g des nach Beispiel 12b hergestellten Diols rührt man 20 Stunden mit 50 ml einer Mischung aus Essigsäure/Wasser/Tetrahydrofuran (65/35/10). Man dampft unter Zusatz von Toluol im Vakuum ein, nimmt den Rückstand in Methylenchlorid auf, schüttelt nacheinander mit Natriumbikarbonatlösung und Sole, trocknet über Magnesiumsulfat und dampft im Vakuum ein. Han erhält 1,6 g des o.a. Ketons als Öl.

IR : 3 600, 3 450 (breit), 2 965, 2 860, 1 738, 976/cm.

12d) (1R, 5S, 6R, 7R)-7-(Tetrahydropyran-2-yloxy)-6-[(E)-3αβ-methyl-3-(tetrahydropyran-2-yloxy)-oct-1-en-6-inyl]-bicyclo[3.3.0]-3-on

Eine Mischung aus 1 g des nach Beispiel 12c hergestellten Ketons, 50 ml Methylenchlorid, 1,5 ml Dihydropyran und 10 mg p-Toluolsulfonsäure rührt man 30 Minuten bei 0-5 °C. Anschließend verdünnt man mit Methylenchlorid, schüttelt mit Natriumbikarbonatlösung und Sole, trocknet über Magnesiumsulfat und dampft im Vakuum ein. Den Rückstand chromatographiert man an Kieselgel mit Hexan/Essigester-Gemischen und erhält 1,25 g des o.a. Bistetrahydropyranyläthers als farbloses Öl.

IR : 2 960, 2 865, 1 738, 978/cm.

Beispiel 13

5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäuremethylester

Eine Lösung von 100 mg 5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure (Herstellung siehe Beispiel 11) in 5 ml Methylenchlorid versetzt man unter Rühren bei 0 °C tropfenweise mit einer ätherischen Diazomethanlösung bis

zur bleibenden Gelbfärbung. Nach Abdampfen des Lösungsmittels reinigt man den Rückstand durch Chromatographie an Kieselgel mit Methylenchlorid/1 % Isopropanol und erhält 90 mg der Titelverbindung als Öl.

IR : 3 600, 2 960, 2 865, 1 735, 978/cm.

Beispiel 14

5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure-tris(hydroxymethyl)amino-methansalz

Zu einer Lösung von 360 mg 5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure (Herstellung siehe Beispiel 11) in 60 ml Acetonitril gibt man bei 65 °C eine Lösung von 121 mg Tris-(hydroxymethyl)m-aminomethan in 0,4 ml Wasser. Unter Rühren läßt man abkühlen, nach 16 Stunden dekantiert man vom Lösungsmittel und trocknet den Rückstand bei 25 °C und 0,1 Torr. Man erhält 320 mg der Titelverbindung als wachsartige Masse.

**Ansprüche**

1. Prostanderivate der allgemeinen Formel I

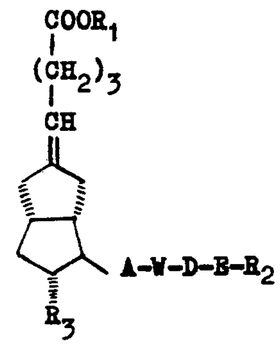

(I)

worin

$R_1$ Wasserstoff, Alkyl mit 1-10 C-Atomen, das durch Halogen, Phenyl, Dimethylamino, Diäthylamino, Methoxy oder Äthoxy substituiert sein kann, Cycloalkyl mit 5 oder 6 C-Atomen, Phenyl, das in 3- oder 4-Stellung durch Halogen, $C_1$-$C_4$-Alkoxy, Hydroxy oder Trifluormethyl substituiert sein kann, oder einen 5- oder 6-gliedrigen heterocyclischen Rest mit mindesten einem Stickstoff-, Sauerstoff- oder Schwefelatom,

A eine $—CH_2$-CH—, trans—CH = CH— oder —C ≡ C—Gruppe,

W eine freie oder an der Hydroxygruppe funktionell abgewandelte Hydroxymethylengruppe oder eine freie oder an der Hydroxygruppe funktionell abgewandelte

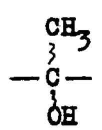 Gruppe

wobei die OH-Gruppe α- oder β-ständig ist,

D und E gemeinsam eine direkte Bindung oder

D eine geradkettige oder verzweigte gesättigte Alkylengruppe mit 1-10 C-Atomen oder eine ungesättigte Alkylengruppe mit bis zu 10 C-Atomen, die gegebenenfalls durch Fluoratome substituiert sind,

E ein Sauerstoffatom oder eine —C ≡ C—Bindung oder eine direkte Bindung,

$R_2$ eine gerad- oder verzweigtkettige, gesättigte oder ungesättigte Alkylgruppe mit 1-7 C-Atomen, eine Cycloalkylgruppe mit 5 oder 6 C-Atomen, eine durch Halogen, Phenyl, $C_1$-$C_4$-Alkyl, Chlormethyl, Fluormethyl, Trifluormethyl, Hydroxy oder Carboxyl substituierte Phenylgruppe oder einen 5- oder 6-gliedrigen heterocyclischen Rest mit mindestens einem Stickstoff-, Sauerstoff- oder Schwefelatom, $R_3$ eine freie oder funktionell abfewandelte Hydroxygruppe,

und falls $R_1$ die Bedeutung eines Wasserstoffatoms hat, deren Salze mit physiologisch verträglichen Basen bedeuten.

2. Verfahren zur Herstellung von Prostanderivaten der im Anspruch 1 angegebenen allgemeinen Formel I, dadurch gekennzeichnet, daß man in an sich bekannter Weise eine Verbindung der allgemeinen Formel II,

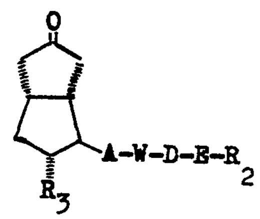

(II)

worin $R_2$, $R_3$, A, W, D und E die im Anspruch 1 angegebenen Bedeutungen aufweisen, gegebenenfalls nach Schutz anwesender freier Hydroxygruppen mit einem Wittig-Reagenz der allgemeinen Formel III

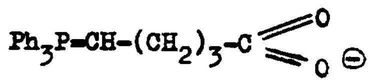

(III)

worin Ph eine Phenylgruppe bedeutet, umsetzt und gegebenenfalls anschließend in beliebiger Reihenfolge Isomere trennt und/oder geschützte Hydroxygruppen freisetzt und/oder freie Hydroxygruppen verestert oder veräthert und/oder eine freie Carboxylgruppe verestert und/oder eine veresterte Carboxylgruppe verseift oder eine Carboxylgruppe mit einer physiologisch verträglichen Base in ein Salz überführt.

3. Arzneimittel, bestehend aus einer oder mehreren Verbindungen gemäß Anspruch 1 und üblichen Hilfs- und Trägerstoffen.

4. 5-{(E)-(1S, 5S, 6R, 7R)-7-Hydroxy-6-[(E)-3α-hydroxy-1-octenyl]-bicyclo[3.3.0]octan-3-yliden}-pentansäure.

5. 5-{(E)-(1S, 5S, 6R, 7R)-7-Hydroxy-6-[(E)-(4RS)-3α-hydroxy-4-methyl-1-octenyl]-bicyclo[3.3.0]octan-3-yliden}-pentansäure.

6. 5-{(E)-(1S, 5S, 6R, 7R)-7-Hydroxy-6-[(E)-(4RS)-4-fluor-3α-hydroxy-1-octenyl]-bicyclo[3.3.0]octan-3-yliden}-pentansäure.

7. 5-{(E)-(1S, 5S, 6R, 7R)-7-Hydroxy-6-[(E)-3αhydroxy-4,4-methylen-1-octenyl]-bicyclo[3.3.0]octan-3-yliden}-pentansäure.

8. 5-{(E)-(1S, 5S, 6R, 7R)-7-Hydroxy-6-[(E)-3α-hydroxy-1-nonenyl]-bicyclo[3.3.0]octan-3-yliden}-pentansäure.

9. 5-{(E)-(1S, 5S, 6R, 7R)-7-Hydroxy-6-[(E)-(4RS)-3α-hydroxy-4-methyl-1-nonenyl]-bicyclo[3.3.0]octan-3-yliden}-pentansäure.

10. 5-{(E)-(1S, 5S, 6R, 7R)-7-Hydroxy-6-[(E)-3α-hydroxy-4-phenyl-1-butenyl]-bicyclo[3.3.0]octan-3-yliden}-pentansäure.

11. 5-{(E)-(1S, 5S, 6R, 7R)-7-Hydroxy-6-[(4RS)-3α-hydroxy-4-methyl-1-octyl]-bicyclo[3.3.0]octan-3-yliden-pentansäure.

12. 5-{(E)-(1S, 5S, 6R, 7R)-7-Hydroxy-6-[3α-hydroxy-1-nonyl]-bicyclo[3.3.0]octan-3-yliden}-pentansäure.

13. 5-{(E)-(1S, 5S, 6R, 7R)-7-Hydroxy-6-[(E)-3α-hydroxy-oct-1-en-6-inyl]-bicyclo[3.3.0]octan-3-yliden}-pentansäure.

14. 5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure.

15. 5-{(E)-(1S, 5S, 6R, 7R)-7-Hydroxy-6-[(6)-3αβ-hydroxy-3-methyl-oct-1-en-6-inyl]-bicyclo[3.3.0]octan-3-yliden}-pentansäure.

16. 5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäuremethylester.

17. 5-{(E)-(1S, 5S, 6R, 7R)-7-Hydroxy-6-[(E)-(4RS)-3α-hydroxy-4-methyl-oct-1-en-6-inyl]-bicyclo[3.3.0]octan-3-yliden}-pentansäure-tris (hydroxymethyl)aminomethansalz.

**Claims**

1. Prostane derivatives of the general formula I

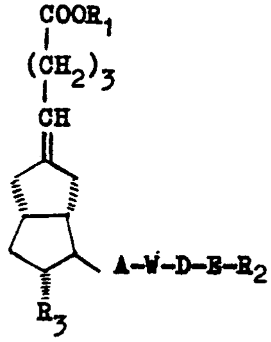

(I)

in which

$R_1$ represents hydrogen ; alkyl having from 1 to 10 carbon atoms which may be substituted by halogen, phenyl, dimethylamino, diethylamino, methoxy or ethoxy ; cycloalkyl having 5 or 6 carbon atoms ; phenyl which may be substituted in the 3- or 4-postion by halogen, alkoxy having from 1 to 4 carbon atoms, hydroxy or trifluoromethyl ; or a 5- or 6-membered heterocyclic radical having at least one nitrogen, oxygen or sulphur atom ;

A represents a $—CH_2—CH_2—$, a trans—CH = CH—, or a —C ≡ C— group,

W represents a free hydroxymethylene group or a hydroxymethylene group functionally modified at the hydroxy group or a free

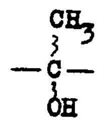

group or a

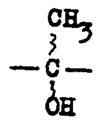

group functionally modified at the hydroxy group, the OH group being in the α- or β- position,

D and E together represent a direct bond, or

D represents a straight-chain or branched saturated alkylene group having from 1 to 10 carbon atoms or an unsaturated alkylene group having up to 10 carbon atoms, each of which is optionally substituted by fluorine atoms,

E represents an oxygen atom or a —C ≡ C— bond or a direct bond,

$R_2$ represents a straight-chain or branched, saturated or unsaturated, alkyl group having from 1 to 7 carbon atoms ; a cycloalkyl group having 5 or 6 carbon atoms ; a phenyl group substituted by halogen, phenyl, alkyl having from 1 to 4 carbon atoms, chloromethyl, fluoromethyl, trifluoromethyl, hydroxy or carboxy ; or a 5- or 6-membered heterocyclic radical having at least one nitrogen, oxygen or sulphur atom ; and

$R_3$ represents a free or functionally modified hydroxy group,

and if $R_1$ has the meaning of a hydrogen atom, the salts thereof with physiologically tolerable bases.

2. Process for the manufacture of prostane derivatives of the general formula I given in claim 1, characterised in that, in a manner known *per se*, a compound of the general formula II

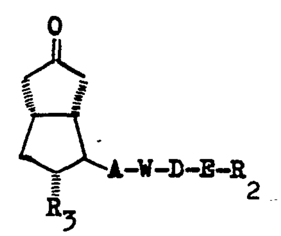

(II)

in which $R_2$, $R_3$, A, W, D and E have the meanings given in claim 1, is reacted, optionally after protecting free hydroxy groups present, with a Wittig reagent of the general formula III

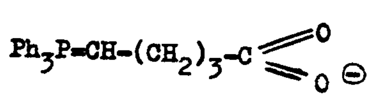

(III)

in which Ph represents a phenyl group, and, if desired, subsequently, in any desired sequence, the isomers are resolved and/or protected hydroxy groups are liberated and/or free hydroxy groups are esterified or etherified and/or a free carboxy group is esterified and/or an esterified carboxy group is hydrolysed or a carboxy group is converted into a salt with a physiologically tolerable base.

3. Medicaments consisting of one or more compounds according to claim 1 and customary auxiliaries and carriers.

4. 5-{(E)-(1S, 5S, 6R, 7R)-7-hydroxy-6-[(E)-3α-hydroxy-1-octenyl]-bicyclo[3,3,0]octan-3-ylidene}-pentanoic acid.

5. 5-{(E)-(1S, 5S, 6R, 7R)-7-hydroxy-6-[(E)-(4RS)-3α-hydroxy-4-methyl-1-octenyl]-bicyclo[3,3,0]octan-3-ylidene}-pentanoic acid.

6. 5-{(E)-(1S, 5S, 6R, 7R)-7-hydroxy-6-[(E)-(4RS)-4-fluoro-3α-hydroxy-1-octenyl]-bicyclo[3,3,0]octan-3-ylidene}-pentanoic acid.

7. 5-{(E)-(1S, 5S, 6R, 7R)-7-hydroxy-6-[(E)-3α-hydroxy 4,4-methylene-1-octenyl]-bicyclo[3,3,3]octan-3-ylidene}-pentanoic acid.

8. 5-{(E)-(1S, 5S, 6R, 7R)-7-hydroxy-6-[(E)-3α-hydroxy-1-nonenyl]-bicyclo[3,3,0]octan-3-ylidene}-pentanoic acid.

9. 5-{(E)-(1S, 5S, 6R, 7R)-7-hydroxy-6-[(E)-3α-hydroxy-4-methyl-1-nonenyl]-bicyclo[3,3,0]octan-3-ylidene}-pentanoic acid.

10. 5-{(E)-(1S, 5S, 6R, 7R)-7-hydroxy-6-[(E)-3α-hydroxy-4-phenyl-1-butenyl]-bicyclo[3,3,0]octan-3-ylidene}-pentanoic acid.

11. 5-{(E)-(1S, 5S, 6R, 7R)-7-hydroxy-6-[(4RS)-3α-hydroxy-4-methyl-1-octyl]-bicyclo[3,3,0]octan-3-ylidene}-pentanoic acid.

12. 5-{(E)-(1S, 5S, 6R, 7R)-7-hydroxy-6-[3α-hydroxy-1-nonyl]-bicyclo[3,3,0]octan-3-ylidene}-pentanoic acid.

13. 5-{(E)-(1S, 5S, 6R, 7R)-7-hydroxy-6-[(E)-3α-hydroxy-oct-1-en-6-ynyl]-bicyclo[3,3,0]octan-3-ylidene}-pentanoic acid.

14. 5-{(E)-(1S, 5S, 6R, 7R)-7-hydroxy-6-[(E)-(4RS)-3α-hydroxy-4-methyloct-1-en-6-ynyl]-bicyclo[3,3,0]octan-3-ylidene}-pentanoic acid.

15. 5-{(E)-(1S, 5S, βR, 7R)-7-hydroxy-6-[(E)-3αβ-hydroxy-3-methyloct-1-en-6-ynyl]-bicyclo[3,3,0]octan-3-ylidene}-pentanoic acid.

16. 5-{(E)-(1S, 5S, 6R, 7R)-7-hydroxy-6-[(E)-(4RS)-3α-hydroxy-4-methyloct-1-en-6-ynyl]-bicyclo[3,3,0]octan-3-ylidene}-pentanoic acid methyl ester.

17. 5-{(E)-(1S, 5S, 6R, 7R)-7-hydroxy-6-[(E)-(4RS)-3α-hydroxy-4-methyloct-1-en-6-ynyl]-bicyclo[3,3,0]octan-3-ylidene}-pentanoic acid, tris (hydroxymethyl) aminomethane salt.

**Revendications**

1. Dérivés du prostane répondant à la formule générale I

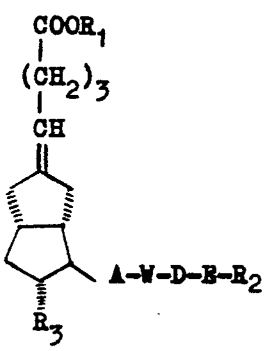

(I)

dans laquelle

$R_1$ représente l'hydrogène, un alkyle contenant de 1 à 10 atomes de carbone, éventuellement porteur d'un halogène ou d'un radical phényle, diméthylamino, diéthylamino, méthoxy ou éthoxy, un cyclo-alkyle à 5 ou 6 atomes de carbone, un phényle portant éventuellement un halogène, un alcoxy en $C_1$-$C_4$, un hydroxy ou un trifluorométhyle en position 3 ou en position 4, ou un radical hétérocyclique pentagonal ou hexagonal qui peut contenir au moins un atome d'azote, d'oxygène ou de soufre,

A représente un radical $—CH_2—CH_2—$, $—CH = CH—$ trans ou $—C \equiv C—$,

W représente un radical hydroxyméthylène libre ou à groupe hydroxy sous la forme d'un dérivé fonctionnel ou un radical

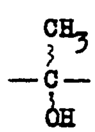

libre ou à groupe hydroxy sous la forme d'un dérivé fonctionnel, le groupe —OH ayant la configuration α ou la configuration β,

D et E représentent ensemble une liaison directe, ou D représente un radical alkylène saturé en $C_1$-$C_{10}$, linéaire ou ramifié, ou un radical alkylène insaturé contenant au plus 10 atomes de carbone, ces radicaux portant éventuellement des atomes de fluor comme substitutants, et E un atome d'oxygène, une liaison $—C \equiv C—$ ou une liaison directe,

$R_2$ représente un alkyle en $C_1$-$C_7$, saturé ou insaturé, linéaire ou ramifié, un cyclo-alkyle en $C_5$ ou $C_6$, un phényle porteur d'un halogène, d'un phényle, d'un alkyle en $C_1$-$C_4$, d'un chlorométhyle, d'un

fluorométhyle, d'un trifluorométhyle, d'un hydroxy ou d'un carboxy, ou un radical hétérocyclique pentagonal ou hexagonal contenant au moins un atome d'azote, d'oxygène ou de soufre, et

$R_3$ représente un groupe hydroxy libre ou sous la forme d'un dérivé fonctionnel,

ainsi que, dans le cas où $R_1$ désigne un atome d'hydrogène, les sels qu'ils forment avec des bases acceptables du point de vue physiologique.

2. Procédé de préparation de dérivés du prostane de formule générale I, selon la revendication 1, procédé caractérisé en ce qu'on fait réagir un composé répondant à la formule générale II

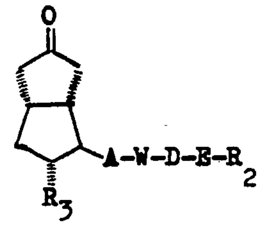

(II)

dans laquelle $R_2$, $R_3$, A, W, D et E ont les significations données à la revendication 1, éventuellement après avoir protégé d'éventuels groupes hydroxy libres, avec un réactif de Wittig répondant à la formule générale III

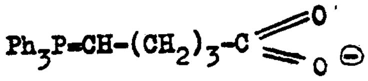

(III)

dans laquelle Rh représente un radical phényle, après quoi, le cas échéant, en opérant dans l'ordre que l'on veut, on sépare des isomères et/ou on libère des groupes hydroxy protégés et/ou estérifie ou éthérifie des groupes hydroxy libres et/ou on estérifie un groupe carboxy libre et/ou on saponifie un groupe carboxy estérifié ou on transforme un groupe carboxy en un sel avec une base acceptable du point de vue physiologique, cela par des méthodes connues.

3. Médicament renfermant un ou plusieurs composés selon la revendication 1 et des additifs et excipients usuels.

4. Acide {hydroxy-7 [hydroxy-3α octène-1 yl-(E)]-6 bicyclo[3.3.0]octylidène-3 (E)-(1S, 5S, 6R, 7R)}-5 pentanoïque.

5. Acide {hydroxy-7 [hydroxy-3α méthyl-4 octène-1 yl-(E)-(4RS)]-6 bicyclo[3.3.0]octylidène-3 (E)-(1S, 5S, 6R, 7R)}-5 pentanoïque.

6. Acide {hydroxy-7 [fluoro-4 hydroxy-3α octène-1 yl-(E)-(4RS)]-6 bicyclo[3.3.0]octylidène-3 (3)-(1S, 5S, 6R, 7R)}-5 pentanoïque.

7. Acide {hydroxy-7 [hydroxy-3α méthylène-4,4 octène-1 yl-(E)]-6 bicyclo[3.3.0]octylidène-3 (E)-(1S, 5S, 6R, 7R)}-5 pentanoïque.

8. Acide {hydroxy-7 [hydroxy-3α nonène-1 yl-(E)]-6 bicyclo[3.3.0]octylidène-3 (E)-(1S, 5S, 6R, 7R)}-5 pentanoïque.

9. Acide {hydroxy-7 [hydroxy-3α méthyl-4 nonène-1 yl-(E)-(4RS)]-6 bicyclo[3.3.0]octylidène-3 (E)-(1S, 5S, 6R, 7R)}-5 pentanoïque.

10. Acide {hydroxy-7 [hydroxy-3α phényl-4 butène-1 yl-(E)]-6 bicyclo[3.3.0]octylidène-3 (E)-(1S, 5S, 6R, 7R)}-5 pentanoïque.

11. Acide {hydroxy-7 [hydroxy-3α méthyl-4 octyl-(4RS)]-6 bicyclo[3.3.0]octylidène-3 (E)-(1S, 5S, 6R, 7R)}-5 pentanoïque.

12. Acide {hydroxy-7 (hydroxy-3α nonyl)-6 bicyclo[3.3.0]octylidène-3 (E)-(1S, 5S, 6R, 7R)}-5 pentanoïque.

13. Acide {hydroxy-7 [hydroxy-3α octène-1 yne-6 yl-(E)]-6 bicyclo[3.3.0]octylidène-3 (E)-(1S, 5S, 6R, 7R)}-5 pentanoïque.

14. Acide {hydroxy-7 [hydroxy-3α méthyl-4 octène-1 yne-6 yl-(E)-(4RS)]-6 bicyclo[3.3.0]octylidène-3 (E)-(1S, 5S, 6R, 7R)}-5 pentanoïque.

15. Acide {hydroxy-7 [hydroxy-3αβ méthyl-3 octène-1 yne-6 yl-(E)]-6 bicyclo[3.3.0]octylidène-3 (E)-(1S, 5S, 6R, 7R)}-5 pentanoïque.

16. Ester méthylique de l'acide {hydroxy-7 [hydroxy-3α méthyl-4 octène-1 yne-6 yl-(E)-(4RS)]-6 bicyclo [3.3.0]octylidène- 3 (E)-(1S, 5S, 6R, 7R)}-5 pentanoïque.

17. Sel formé par l'acide {hydroxy-7 [hydroxy-3α méthyl-4 octène-1 yne-6 yl-(E)-(4RS)]-6 bicyclo [3.3.0]octylidène-3 (E)-(1S, 5S, 6R, 7R)}-5 pentanoïque et l'amino-tris-(hydroxyméthyl)-méthane.